# EUROPÄISCHE PATENTANMELDUNG

(11) **EP 0 705 836 A1**
(43) Veröffentlichungstag der Anmeldung: **10.04.1996**
(21) Anmeldenummer: 95109778.1
(22) Anmeldetag: 23.06.1995
(51) Int. Cl.: C07D 498/10, C07F 7/18, C07F 9/145, C07F 9/6574

(54) **Polyalkylpiperidin-Verbindungen**

(30) Priorität: 01.07.1994 DE 4423055
(71) Anmelder: HOECHST AKTIENGESELLSCHAFT, D-65929 Frankfurt am Main (DE)
(72) Erfinder: Gaa, Karl, Dr., D-89349 Burtenbach (DE); Zäh, Matthias, Dr., D-86368 Gersthofen (DE); Wiedemann, Josef, Dr., D-86441 Zusmarshausen (DE)

(57) **Zusammenfassung**

Polyalkylpiperidin-Verbindungen der Formel I 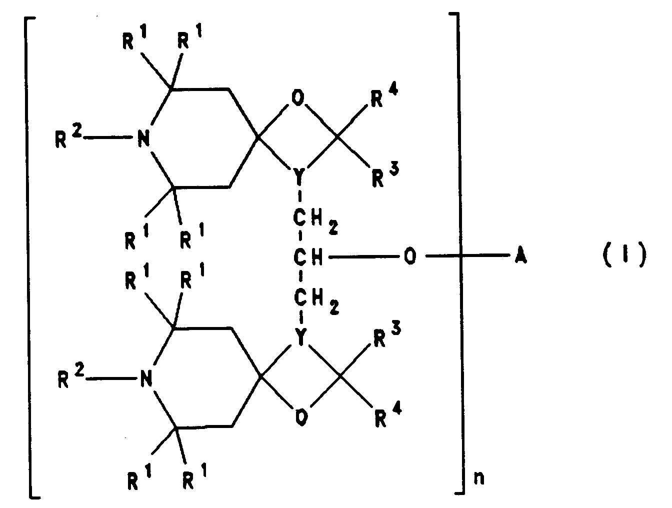
worin Y eine der Gruppen 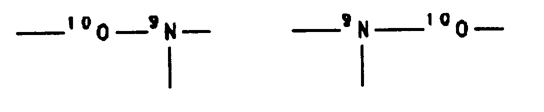
ist, sind sehr wirksame Stabilisatoren für organische Stoffe.

## Beschreibung

Die vorliegende Erfindung bezieht sich auf neue Polyalkylpiperidin-Verbindungen, die in fast allen Eigenschaften Verbesserungen zeigen.

Es ist bekannt, daß organische Verbindungen durch Licht, Strahlung, Sauerstoff oder Wärme geschädigt werden. Es gibt bereits viele Druckschriften, die Verbindungen zur Stabilisierung von organischem Material beschreiben. Ein Teil davon betrifft Verbindungenauf der Basis des 2,2,6,6-Tetramethylpiperidins. Dabei haben sich Spiro-Verbindungen als besonders wirksam erwiesen (vgl. EP 8 102, EP 28 318, EP 280 263).

Weiterhin sind Diazaspirodecane der Formel 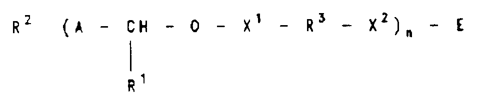
worin R¹ eine Diazaspirodecan-Gruppedarstellt, bekannt (vgl. EP 57 885). As Nachteil dieser Verbindungen hat sich herausgestellt, daß sie in der Wärme relativ flüchtig sind

Auf der Suche nach weiter verbesserten Produkteigenschaften wurde gefunden, daß durch Verbindungen ähnlicher Struktur unerwarteterweise das gesamte Eigenschaftsprofil verbessert wird.

Die Erfindung betrifft somit Verbindungen der Formel I 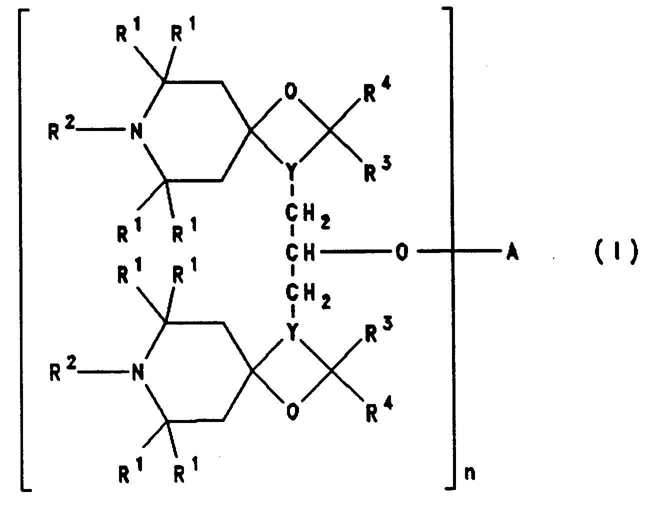
worin Y eine der Gruppen 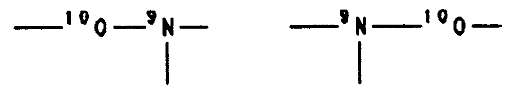
ist,
R¹ ein Wasserstoffatom oder eine Alkylgruppe bedeutet,
R² eine Gruppe der Formel R¹⁷-O- oder R¹⁸-CO- (Acyl) ist,
R³ und R⁴ gleich oder verschieden sind und ein Wasserstoffatom oder eine Alkylgruppe bedeuten, oder R³ und R⁴ zusammen mit dem sie verbindenden C-Atom einen Ring mit 5 bis 12 Ringliedern oder eine Gruppe der Formel 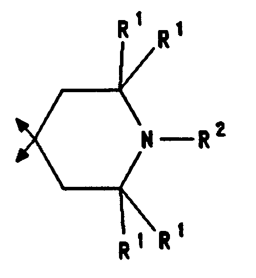
bilden,
n = 1, 2 oder 3 bedeutet und
A bei n = 1
ein Wasserstoffatom, R⁵-, R⁵-CO-, R⁵-NH-CO- (Urethan), -COOR⁵, -P(OR⁶)(OR⁷), -Si(OR⁵)(OR⁵)(OR⁵), -SiR⁵R⁵R⁵ oder die Gruppe 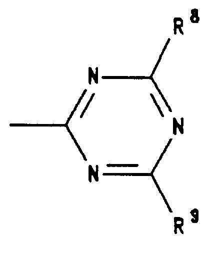
A bei n = 2
-R¹¹-, -CO-R¹²-CO-, -CO-NH-R¹³-NH-CO-, -CO-, = POR⁵, = Si(OR⁵)(OR⁵), = SiR⁵R⁵ oder die Gruppe 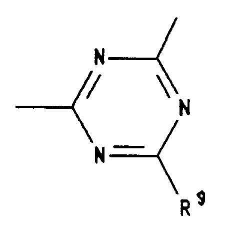
A bei n = 3
R¹⁴≡, R¹⁵(CO-)₃, R¹⁶(NH-CO-)₃ ≡Si(OR⁵), ≡SiR⁵ oder die Gruppe 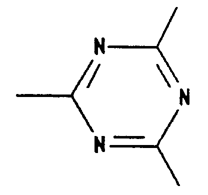
bedeutet,
R⁵ eine C₁-C₁₈-Alkylgruppe, eine C₂-C₁₈-Alkenylgruppe, eine C₂-C₁₈-Alkinylgruppe, eine C₅-C₁₂-Cycloalkylgruppe, eine C₆-C₁₀-Bicycloalkylgruppe, eine C₅-C₈-Cycloalkenylgruppe, eine C₆-C₁₀-Arylgruppe, eine C₇-C₉-Aralkylgruppe, eine C₇-C₉-Alkarylgruppe, gegebenenfalls substituiert durch C₁-C₄-Alkyl oder Phenyl, bedeutet, wobei mehrere Reste R⁵ gleich oder verschieden sein können, R⁶ und R⁷ gleich oder verschieden sind und eine C₁-C₁₈-Alkylgruppe, eine C₂-C₁₈-Alkenylgruppe, eine C₂-C₁₈-Alkinylgruppe, eine C₅-C₁₂-Cycloalkylgruppe, eine C₈-C₁₀-Bicycloalkylgruppe, eine C₅-C₈-Cycloalkenylgruppe, eine C₈-C₁₀-Arylgruppe, eine C₇-C₉-Aralkylgruppe oder eine C₇-C₉-Aralkylgruppe, substituiert durch C₁-C₄ -Alkyl oder Phenyl, bedeuten, oder ein Rest sind der Formel II oder III 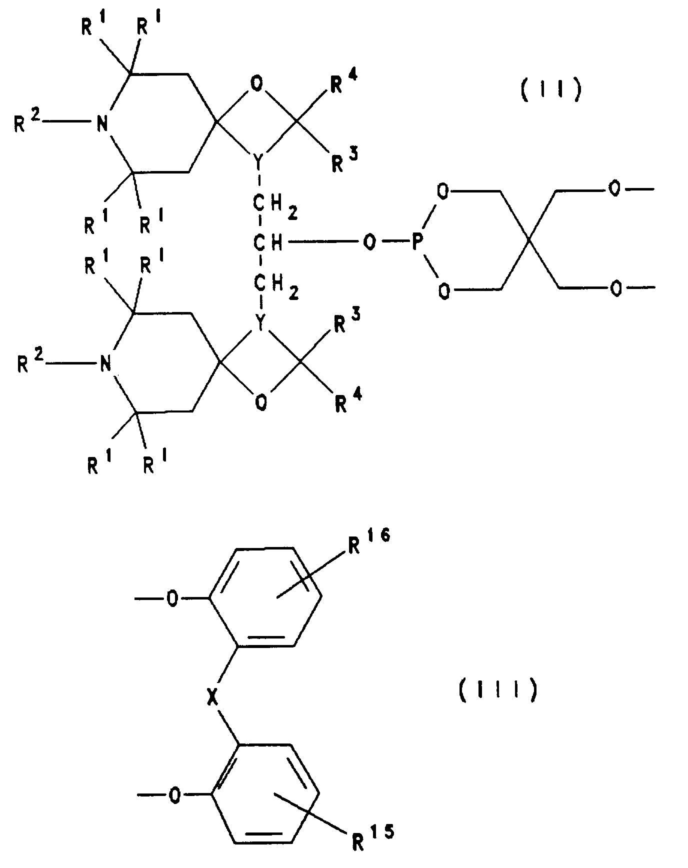
worin R¹, R², R³, R⁴ und R⁵ die obengenannte Bedeutung haben,
und X eine direkte Bindung oder Methylengruppe oder eine 1,1-Alkylidengruppe mit 2 bis 5 C-Atomen bedeutet,
R⁸ und R⁹ gleich oder verschieden sind und -OR⁵, NHR⁵, -NR¹⁷R¹⁸, oder eine der beiden Gruppen 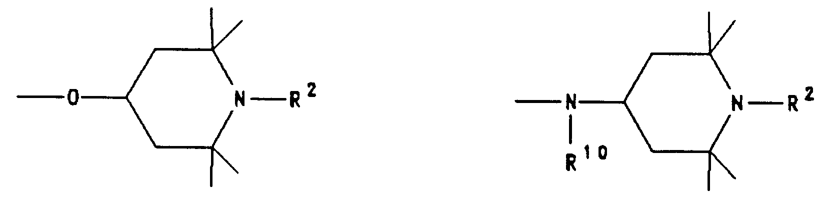
worin R¹⁰ ein Wasserstoffatom, eine C₁-C₁₂-Alkylgruppe, eine C₅-C₇-Cycloalkylgruppe, eine C₇-C₉-Aralkylgruppe, eine C₈-C₁₈-Alkanoylgruppe, eine C₃-C₅-Alkenoylgruppe oder eine Benzoylgruppe bedeutet, oder 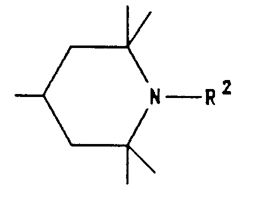
ist,
R¹¹, R¹² und R¹³ gleich oder verschieden sind und eine C₁-C₁₂-Alkylengruppe, eine C₅-C₁₃-Cycloalkylengruppe, eine C₈-C₁₀-Bicycloalkylengruppe, eine C₆-C₁₀-Arylengruppe, eine C₇-C₉-Aralkylengruppe oder C₇-C₉-Arylengruppe, substituiert durch C₁-C₄-Alkyl oder Phenyl bedeuten,
R¹⁴, R¹⁵ und R¹⁶ gleich oder verschieden sind und einen dreifach funktionellen C₃-C₁₈-Alkyl-, C₅-C₇-Cycloalkyl-, C₆-C₁₀-Aryl- oder C₆-C₁₀-Arylrest, substituiert durch C₁-C₄-Alkyl oder Phenyl bedeuten,
R¹⁷ und R¹⁸ gleich oder verschieden sind und eine C₁-C₁₀-Alkylgruppe, eine C₅-C₇-Cycloalkylgruppe, eine C₆-C₁₀-Arylgruppe bedeuten, oder R¹⁷ und R¹⁸ zusammen mit dem Stickstoff einen 5- bis 12-gliedrigen Ring bilden, der auch noch Sauerstoff enthalten kann.

Die Erfindung bezieht sich auch auf die Herstellung dieser Verbindungen sowie
die Verwendung zum Stabilisieren von organischem Material, insbesondere von Kunststoffen, Ölen und Lacken.

In der Formel I 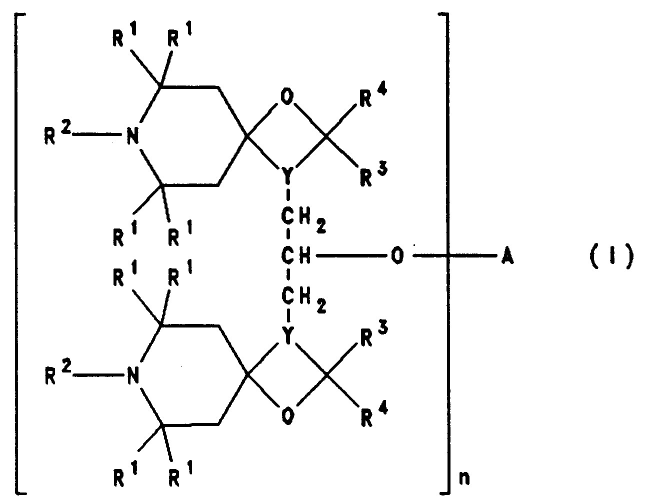
ist Y eine der Gruppen 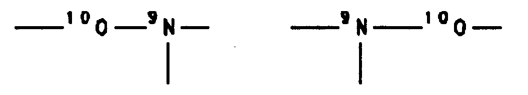
wobei die angegebenen Ziffern die Stellung der Atome im Spirodecan-Fünfring bezeichnen. Bevorzugt ist Y eine Gruppierung, bei welcher das Sauerstoffatom dem die beiden Ringe gemeinsamen C-Atom benachbart ist, das heißt, 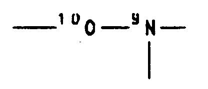
n ist 1, 2 oder 3, vorzugsweise 1.
R¹ ist ein Wasserstoffatom oder eine C₁-C₁₈-Alkylgruppe, vorzugsweise Methylgruppe.
R² ist eine der Gruppen R¹⁷-O- oder R¹⁸-CO- (Acyl), worin R¹⁷ und R¹⁸ gleich oder verschieden sind und eine C₁-C₁₈-, vorzugsweise C₁-C₈-Alkylgruppe, eine C₅-C₇ -Cycloalkylgruppe, eine C₆-C₁₀-, vorzugsweise C₆-Arylgruppe, oder R¹⁷ und R¹⁸ zusammen mit dem Stickstoff einen 5- bis 12-, vorzugsweise 5- bis 6-gliedrigen Ring bilden, der auch noch Sauerstoff enthalten kann, insbesondere einen Pyrrolidin-, Piperidin- oder Morpholinring.
R³ und R⁴ sind gleich oder verschieden und bedeuten ein Wasserstoffatom, eine C₁-C₁₈-, vorzugsweise C₁-C₈-Alkylgruppe oder R³ und R⁴ bilden zusammen mit dem sie verbindenden C-Atom einen Ring mit 5 bis 12, vorzugsweise mit 6 oder 12 Ringgliedern, oder bilden zusammen mit dem sie verbindenden C-Atom eine Gruppe 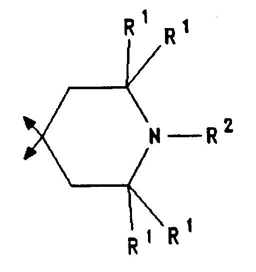
Bei n = 1 ist A ein Wasserstoffatom, eine Gruppe R⁵-, R⁵-CO-, R⁵-NH-CO-(Urethan),-COOR⁵, -P(OR⁶)(OR⁷), -Si(OR⁵)(OR⁵)(OR⁵) oder -SiR⁵R⁵R⁵, worin R⁵ die obengenannte Bedeutung hat.
R⁵ bedeutet eine C₁-C₁₈-, vorzugsweise C₁-C₈-Alkylgruppe, eine C₂-C₁₈-vorzugsweise C₃-C₆-Alkenylgruppe, eine C₂-C₁₈-, vorzugsweise C₂-C₈-Alkinylgruppe, eine C₅-C₁₂-, vorzugsweise C₅-C₆-Cycloalkylgruppe, eine C₆-C₁₀ vorzugsweise C₆-C₁₀-Bicycloalkylgruppe, eine C₅-C₈-, vorzugsweise C₅-C₈-Cycloalkenylgruppe, eine C₆-C₁₀-, vorzugsweise C₆- oder C₁₀-Arylgruppe, eine C₇-C₉-Aralkylgruppe, eine C₇-C₉-Aralkylgruppe, substituiert durch C₁-C₄-Alkyl oder Phenyl, wobei mehrere Reste R⁵ gleich oder verschieden sein können.
R⁶ und R⁷ sind gleich oder verschieden und bedeuten eine C₁-C₁₈-, vorzugsweise C₁-C₈-Alkylgruppe, eine C₂-C₁₈-, vorzugsweise C₃-C₆-Alkenylgruppe, eine C₂-C₁₈-, vorzugsweise C₂-C₈-Alkinylgruppe, eine C₅-C₁₂-, vorzugsweise C₅-C₆-Cycloalkylgruppe, eine C₆-C₁₀-, vorzugsweise C₈-C₁₀-Bicycloalkylgruppe, eine C₅-C₈-, vorzugsweise C₅-C₆-Cycloalkenylgruppe, eine C₆-C₁₀-, vorzugsweise C₆-oder C₁₀-Arylgruppe, eine C₇-C₉-Aralkylgruppe oder eine C₇-C₉-Aralkylgruppe, substituiert durch C₁-C₄-Alkyl oder Phenyl, bedeuten, oder sind ein Rest der Formel II oder III 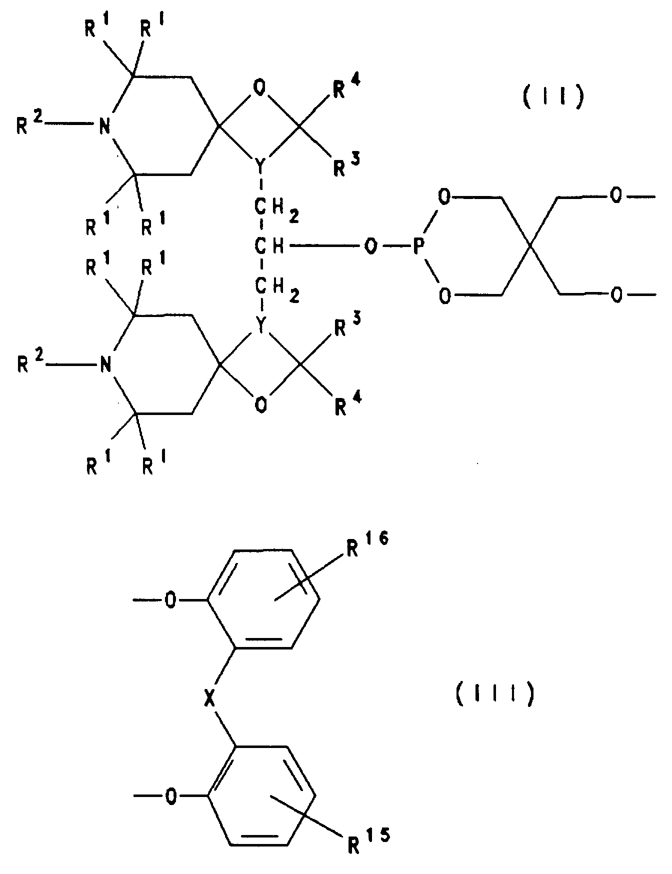
worin R¹, R², R³, R⁴ und R⁵ die obengenannte Bedeutung haben, und X eine direkte Bindung oder Methylengruppe oder eine 1,1-Alkylidengruppe mit 2 bis 5 C-Atomen bedeutet.

Oder A ist 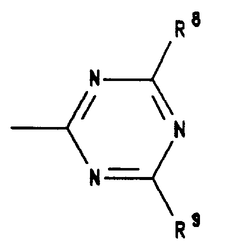
worin R⁸ und R⁹ gleich oder verschieden sind und -OR⁵, -NHR⁵, -NR¹⁷R¹⁸, oder eine der beiden Gruppen 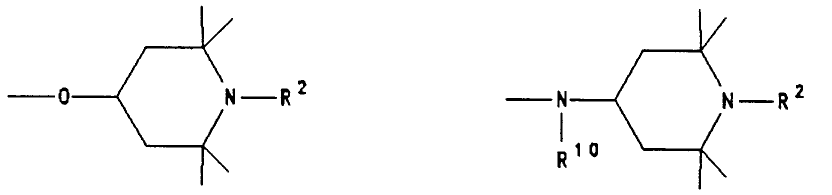
bedeuten, worin R¹⁰ ein Wasserstoffatom, eine C₁-C₁₂-, vorzugsweise C₁-C₈-Alkylgruppe, eine C₅-C₇-, vorzugsweise C₅-C₆-Cycloalkylgruppe, eine C₇-C₉-Aralkylgruppe, eine C₈-C₁₈-, vorzugsweise C₂-C₈-Alkanoylgruppe, eine C₃-C₅-, vorzugsweise C₃-C₄-Alkenoylgruppe oder eine Benzoylgruppe bedeutet, oder 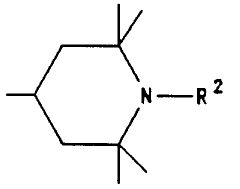
ist. Vorzugsweise ist A ein Wasserstoffatom, R⁵- oder R⁵-CO-, wobei R⁵
die obengenannte Bedeutung hat, insbesondere C₁- bis C₁₈-Acyl, speziell Formyl, Acetyl, Lauryl, Stearyl.

Bei n = 2 ist A -R¹¹-, -CO-R¹²-CO-, -CO-NH-R¹³-NH-CO-, -CO-, =POR⁵, = Si(OR⁵)(OR⁵), = SiR⁵R⁵R⁵ oder die Gruppe 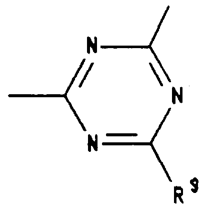
worin R⁵ und R⁹ die obengenannte Bedeutung haben und
R¹¹, R¹² und R¹³ gleich oder verschieden sind und eine C₁-C₁₂-, vorzugsweise C₁-C₈ -Alkylengruppe, speziell eine Methylen-, Ethylen-, Butylen-, Hexamethylen-oder Octamethylengruppe, eine C₅-C₁₃-, vorzugsweise C₅-C₆-Cycloalkylengruppe, sspeziell eine Cyclohexylen-, 4,4'-Dicyclohexylmethylen oder Isophorongruppe, eine C₈-C₁₀-, vorzugsweise C₁₀-Bicycloalkylengruppe, eine C₆-C₁₀-, vorzugsweise C₆-oder C₁₀-Arylengruppe, speziell Phenylen- oder Naphthylengruppe, eine C₇-C₉-Aralkylengruppe oder C₇-C₉-Arylengruppe, substituiert durch C₁-C₄-Alkyl oder Phenyl, insbesondere Toluylen, oder eine 4,4'-Diphenylmethangruppe bedeuten.

Bei n = 3 ist A R¹⁴≡, R¹⁵(CO-)₃, R¹⁶(NH-CO-)₃, ≡Si(OR⁵), ≡SiR⁵ oder die Gruppe 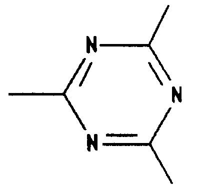
wobei R¹⁴, R¹⁵ und R¹⁶ gleich oder verschieden sind und einen dreifach funktionellen C₃-C₁₈-, vorzugsweise C₃-C₈-Alkyl-, C₅-C₇-, vorzugsweise C₅-C₆-Cycloalkyl-, C₆-C₁₀-, vorzugsweise C₆- oder C₁₀-Aryl- oder C₆-C₁₀-Arylrest, substituiert durch C₁-C₄-Alkyl oder Phenyl, bedeuten.

Die Herstellung der Verbindungen der Formel I mit n = 1 und A = H erfolgt entweder durch die Verknüpfung von Verbindungen der Formel IV 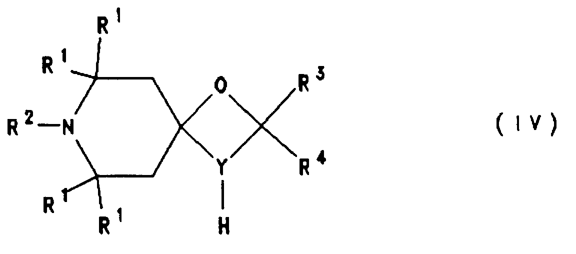
mit Epichlorhydrin, oder durch die Umsetzung von Verbindungen der Formel V 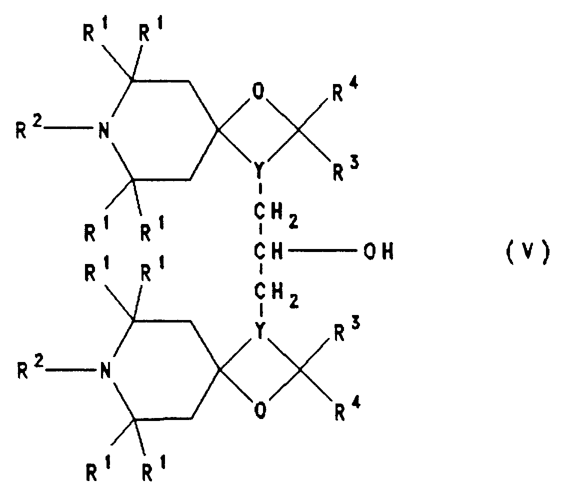
mit den üblichen Reagenzien zu den Verbindungen der Formel I mit dem gewünschten R².

Die Verbindungen der Formel I mit A nicht H lassen sich vorteilhafterweise durch an sich bekannte Umsetzungen aus den Alkoholen der Formel V mit den entsprechenden reaktiven Komponenten der Formel VI

A⁅Z]ₙ (VI)

herstellen, wobei
Z ein Halogenatom oder die Gruppe = OR¹⁷ bedeutet für A = R⁵-CO-, -CO-R¹²-CO-, R¹⁵(CO-)₃, -CO-, -COOR⁵, -P(OR⁶)(OR⁷), =POR⁵;
Z ein Halogenatom ist für A = R⁵-, -R¹¹-, -Si(OR⁵)(OR⁵)(OR⁵), =Si(OR⁵)(OR⁵), ≡Si(OR⁵), -SiR⁵R⁵R⁵, =SiR⁵R⁵, ≡SiR⁵, Triazinderivat;
und Z die Gruppe -NCO ist für A = R⁵-, -R¹³- oder R¹⁶≡.

Eine weitere Möglichkeit zur Herstellung der erfindungsgemäßen Verbindungen der Formel I mit A nicht H besteht darin, die Verbindungen I mit R² = H und A nicht H mit den üblichen Reagenzien zu den substituierten Produkten 1 mit R² nicht H umzusetzen.

Die Umsetzung erfolgt in einem protischen oder aprotischen, organischen Löse-mittel, vorzugsweise einem Kohlenwasserstoff, insbesondere einem aromatischen Kohlenwasserstoff wie beispielsweise Toluol, Xylol oder in Gemischen hieraus oder in einem Alkohol, vorzugsweise einem aliphatischen Alkohol, insbesondere in einem C₁-C₁₂-Alkohol, wie in Isopropanol oder in einer Mischung aus einem Kohlenwasserstoff und einem Alkohol, insbesondere Xylol und Isopropanol. Eine weitere Möglichkeit besteht darin, eine der Reaktionskomponenten im Überschuß als Lösemittel zu verwenden.

Wenn bei der Einführung von A
- Halogenwasserstoff abgespalten wird, so erfolgt die Umsetzung in Gegenwart von Basen;
- Alkohol abgespalten wird, so erfolgt die Umsetzung in Gegenwart von katalytischen Mengen Basen oder in Gegenwart der üblichen Umesterungs-katalysatoren;
- ein Isocyanat addiert wird, kann die Umsetzung ohne Zusätze erfolgen.

Die Umsetzung erfolgt bei einer Temperatur zwischen 20°C und dem Siedepunkt des Lösemittels, die geeignete Temperatur ist abhängig von der verwendeten Base und von der Reaktivität der eingesetzten Verbindung der Formel VI.

Die erfindungsgemäßen Verbindungen der Formel I können in Form der freien Basen oder als Säureadditionssalzvorliegen. Geeignete Anionen stammen beispielsweise von anorganischen Säuren und insbesondere von organischen Säuren oder Sulfonsäuren. Als anorganische Anionen sind beispielsweise Chlorid, Bromid, Sulfat, Tetrafluoroborat, Phosphat und Rhodanid zu nennen. Als Carbonsäuren kommen Format, Acetat, Propionat, Hexanoat, Cyclohexanoat, Lactat, Stearat, Acrylat, Methacrylat, Citrat, Malonat oder Succinat sowie Anionen von Polycarbonsäuren mit bis zu 3000 COOH-Gruppen in Betracht. Sulfonsäure-Anionen sind beispielsweise Benzolsulfonat oder Tosylat.

Die erfindungsgemäßen Verbindungen eignen sich in hervorragender Weise zum Stabilisieren von organischen Material gegen die Einwirkung von Licht, Sauerstoff und Wärme. Sie werden dem zu stabilisierenden organischen Material in einer Konzentration von 0.001 bis 5 Gew.-%, vorzugsweise von 0.02 bis 1 Gew.-%, bezogen auf das organische Material vor, während oder nach seiner Herstellung zugesetzt.

Unter organischem Material sind beispielsweise Vorprodukte für Kunststoffe, Lacke und Öle, insbesondere jedoch Kunststoffe, Lacke und Öle selbst, zu verstehen.

Gegenstand der vorliegenden Erfindung ist außerdem gegen die Einwirkung von Licht, Sauerstoff und Wärme stabilisiertes organisches Material, insbesondere Kunststoffe, Lacke und Öle, welches die Verbindung in den oben angegebenen Konzentrationen enthält. Zu diesen organischen Materialien gehören folgende Stoffe:
1. Polymere von Mono- und Diolefinen, beispielsweise Polyethylen hoher, mittlerer oder niederer Dichte (das gegebenenfalls vernetzt sein kann), Polypropylen, Polyisobutylen, Polybuten-1, Polymethylpenten-1, Polyisopren oder Polybutadien sowie Polymerisate von Cycloolefinen, wie beispielsweise von Cyclopenten oder Norbornen.
2. Mischungen der unter 1) genannten Polymeren, beispielsweise Mischungen von Polypropylen mit Polyethylen oder mit Polyisobutylen.
3. Copolymere von Mono- und Diolefinen untereinander oder mit anderen Vinylmonomeren, wie beispielsweise Ethylen-Propylen-Copolymere, Propylen-Butent-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.
4. Polystyrol, Poly(p-methylstyrol).
5. Copolymere von Styrol oder α-Methylstyrol mit Dienen oder Acrylderivaten, wie beispielsweise Styrol-Butadien, Styrol-Maleinsäureanhydrid, Styrol-Acrylnitril, Styrol-Ethylmethacrylat, Styrol-Butadien-Ethylacrylat, Styrol-Acrylnitril-Methacrylat; Mischungen von hoher Schlagzähigkeit aus Styrol-Copolymeren und einem anderen Polymer, wie beispielsweise einem Polyacrylat, einem Dien-Polymeren oder einem Ethylen-Propylen-Dien-Terpolymeren; sowie Block-Copolymere des Styrols, wie beispielsweise Styrol-Butadien-Styrol, Styrol-Isopren-Styrol, Styrol-Ethylen/Butylen-Styrol oder Styrol-Ethylen/Propylen-Styrol.
6. Pfropfcopolymerevon Styrol, wie beispielsweise Styrol auf Polybutadien, Styrol und Acrylnitril auf Polybutadien, Styrol und Maleinsäureanhydrid auf Polybutadien, Styrol und Alkylacrylate oder Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, die beispielsweise als sogenannte ABS-, MBS-, ASA- oder AES-Polymere bekannt sind.
7. Polyvinylchlorid.
8. Mischpolymerisate des Vinylchlorids, welche durch die bekannten Verfahren hergestellt werden können (beispielsweise Suspensions-, Masse- oder Emulsionspolymerisation).
9. Mischpolymere des Vinylchlorids mit bis zu 30 Gew.-% an Comonomeren, wie beispielsweise Vinylacetat, Vinylidenchlorid, Vinylether, Acrylnitril, Acrylsäureester, Maleinsäuremono- oder -diester oder Olefinen, sowie Pfropfpolymerisate des Vinylchlorids.
10. Halogenhaltige Polymere, wie beispielsweise Polychloropren, Chlorkautschuk, chloriertes oder chlorsulfoniertes Polyethylen, Epichlorhydrin-homo und - copolymere, insbesondere Polymere aus halogenhaltigen Vinylverbindungen, wie beispielsweise, Polyvinylidenchlorid, Polyvinylfluorid, Polyvinylidenfluorid; sowie deren Copolymere wie von Vinylchlorid-Vinylidenchlorid, Vinylchlorid-Vinylacetat oder Vinylidenchlorid-Vinylacetat.
11. Polymere, die sich von α-β-ungesättigten Säuren und deren Derivaten ableiten, wie Polyacrylate und Polymethacrylate, Polyacrylamide und Polyacrylnitrile.
12. Copolymere der unter 11) genannten Monomeren untereinander oder mit anderen ungesättigten Monomeren, wie beispielsweise Acrylnitril-Butadien-Copolymere, Acrylnitril-Alkylacrylat-Copolymere, Acrylnitril-Alkoxyacrylat-Copolymere, Acrylnitril-Vinylhalogenid-Copolymere oder Acrylnitril-Alkylmethacrylat-Butadien-Copolymere.
13. Polymere, die sich von ungesättigten Alkoholen und Aminen bzw. deren Acylderivaten oder Acetalen ableiten, wie Polyvinylalkohol, Polyvinylacetat, - stearat, - benzoat, -maleat, Polyvinylbutyral, Polyallylphthalat, Polyallylmelamin.
14. Homo- und Copolymere von cyclischen Ethern, wie Polyethylenglykole, Polyethylenoxyd, Polypropylenoxyd oder deren Copolymere mit Bisglycidylethern.
15. Polyacetale, wie Polyoxymethylen, sowie solche Polyoxymethylene, die Comonomere wie beispielsweise Ethylenoxyd enthalten.
16. Polyphenylenoxyde und -sulfide und deren Mischungen mit Styrolpolymeren.
17. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte (Polyisocyanate-Polyole-Prepolymere).
18. Polyamide und Copolyamide, die sich von Diaminen und Dicarbonsäuren und/oder von Aminocarbonsäuren oder den entsprechenden Lactamen ableiten, wie Polyamid-4, Polyamid-6, Polyamid-6.6, Polyamid-6.10, Polyamid-11,
   Polyamid-12, Poly-2,4,4-trimethylhexamethylenterephthalamid, Poly-m-phenylenisophthalamid, sowie deren Copolymere mit Polyethern, wie beispielsweise mit Polyethylenglykol, Polypropylenglykol oder Polytetramethylenglykol.
19. Polyharnstoffe, Polyimide und Polyamid-imide.
20. Polyester, die sich von Dicarbonsäuren und Diolen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-(2,2-bis-(4-hydroxyphenyl)-propan)-terephthalat, Polyhydroxybenzoate, sowie Block-Polyether-ester, die sich von Polyethylen mit Hydroxyendgruppen, Dialkoholen und Dicarbonsäuren ableiten.
21. Polycarbonate und Polyestercarbonate.
22. Polysulfone, Polyethersulfone und Polyetherketone.
23. Vernetzte Polymere, die sich von Aldehyden einerseits und Phenolen, Harnstoff oder Melaminandererseits ableiten, wie Phenol-Formaldehyd-, Harnstoff-Formaldehyd- und Melamin-Formaldehydharze.
24. Trocknende und nicht trocknende Alkydharze.
25. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
26. Vernetzbare Acrylharze, die sich von substituierten Acrylsäureestern ableiten, wie beispielsweise Epoxyacrylaten, Urethan-acrylaten oder Polyester-acrylaten.
27. Alkydharze, Polyesterharze und Acrylatharze, die mit Melaminharzen, Harnstoffharzen, Polyisocyanaten oder Epoxidharzen vernetzt sind.
28. Vernetzbare Epoxidharze, die sich von Polyepoxiden ableiten, beispielsweise von Bis-glycidylethern oder von cycloaliphatischen Diepoxiden.
29. Natürliche Polymere, wie Cellulose, Naturkautschuk, Gelatine sowie deren polymerhomolog chemisch abgewandelte Derivate, wie Celluloseacetate, -propionate und -butyrate, oder Celluloseether, wie Methylcellulose.
30. Mischungen der oben erwähnten Polymeren, wie beispielsweise PP/EPDM, Polyamid-6/EPDM oder ABS, PVC/EVA, PVC/ABS, PVC/MBS, PC/ABS, PBTP/ABS, PC/ASA, PC/PBT, PVC/CPE, PVD/Acrylat, POM/thermoplastisches PUR, POM/Acrylat, POM/MBS, PPE/HIPS, PPE/Polyamid-6.6 und Copolymere, PA/HDPE, PA/PP, PA/PPE.
31. Natürlich vorkommende und synthetische organische Stoffe, welche reine Monomere oder Mischungen von Monomeren sind, wie beispielsweise Mineralöle, tierische und pflanzliche Fette, Öle und Wachse, oder Öle, Fette und Wachse auf Basis synthetischer Ester oder Mischungen dieser Stoffe.
32. Wäßrige Dispersionen von Natur- oder Synthesekautschuk.

Das durch die erfindungsgemäßen Verbindungen stabilisierte organische Material kann gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Verbindungen zugesetzt werden können, sind beispielsweise Verbindungen auf der Basis sterisch gehinderter Amine oder sterisch gehinderter Phenole oder Schwefel oder Phosphor enthaltende Costabilisatoren.

Als derartige Verbindungen kommen beispielsweise in Frage:
1. Antioxidantien
   1.1 Alkylierte Monophenole, beispielsweise 2,6Di-t-butyl-4-methylphenol, 2-Butyl-4,6-dimethylphenol, 2,6-Di-t-butyl-4-ethylphenol, 2,6-Di-t-butyl-4-n-butylphenol, 2,6-Di-t-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-t-butyl-4-methoxymethylphenol, 2,6-Di-nonyl-4-methylphenol, 2,4-Dimethyl-6(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen dieser Verbindungen.
   1.2 Alkylthiomethylphenole, beispielsweise 2,4-Di-octylthiomethyl-6-t-butylphenol, 2,4-Dioctylthiomethyl-6-methylpheno, 2,4-Di-octylthiomethyl-6-ethylpheno, 2,6-Didodecylthiomethyl-4-nonylphenol.
   1.3 Hydrochinone und alkylierte Hydrochinone, beispielsweise 2,6-Di-t-butyl-4-methoxyphenol, 2,5-Di-t-butyl-hydrochinon, 2,5-di-t-amyl-hydrochinon, 2,6-Diphenyl-4-octadecyloxyphenol, 2,6-Di-t-butyl-hydrochinon, 2,5-Di-t-butyl-4-hydroxyanisol, 3,5-Di-t-butyl-4-hydroxyanisol, 3,5-Di-t-butyl-4-hydroxyphenol-stearat, Bis-(3,5-di-t-butyl-4-hydroxyphenyl)adipat.
   1.4 Hydroxylierte Thiodiphenylether, beispielsweise 2,2'-Thio-bis-(6-t-butyl-4-methylphenol),2,2'-Thio-bis-(4-octylphenol),4,4'-Thio-bis-(6-t-butyl-3-methylphenol), 4,4'-Thio-bis-(6-t-butyl-2-methyl-phenol),4,4'-Thio-bis-(3,6-di-sec.-amylphenol),4,4'-Bis-(2,6-dimethyl-4-hydroxyphenyl)-disulfid.
   1.5Alkyliden-Bisphenole,beispielsweise2,2'-Methylen-bis-(6-t-butyly-4-methylphenol), 2,2'-Methylen-bis-(6-t-butyl-4-ethylphenol), 2,2'-Methylen-bis-[4-methyl-6-(α,-methylcyclohexyl)-phenol], 2,2'-Methylen-bis-(4-methyl-6-cyclohexylphenol), 2,2'-Methylenbis-(6-nonyl-4-methylphenol),2,2'-Methylen-bis-(4,6-di-t-butylphenol),2,2'-Ethyliden-bis-(4,6-di-t-butylphenol), 2,2'-Ethyliden-bis-(6-t-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-t-butyphenol), 4,4'-Methylen-bis-(6-t-butyl-2-methylphenol), 1,1-Bis-(5-t-butyl-4-hydroxy-2-methypheny)-butan, 2,6-Bis-(3-t-butyl-5-methyl-2-hydroxybenzyl)-4-methyphenol, 1,1,3-Tris-(5-t-butyl-4-hydroxy-2-methyl-phenyl)-butan, 1,1-Bis-(5-t-butyl-4-hydroxy-2-methylpheny)-3-n-dodecylmercaptobutan, Bis-(3-t-butyl-4-hydroxy-5-methyl-phenyl)-dicyclopentadien, Bis-[2-(3'-t-butyl-2'-hydroxy-5'-methyl-benzyl)-6-t-butyl-4-methylphenyl]-terephthalat, 1,1-Bis-(3,5-dimethyl-2-hydroxyphenyl)-butan,2,2-Bis-(3,5-di-t-butyl-4-hydroxyphenyl)-propan, 2,2-Bis-(5-t-butyl-4-hydroxy-2-methylphenyl)-4-n-dodecylmercaptobutan, 1,1,5,5-Tetra-(5-t-butyl-4-hydroxy-2-methylphenyl)-pentan.
   1.6 O-, N- und S-Benzylverbindungen, beispielsweise 3,5,3',5'-Tetra-t-butyl-4,4'-dihydroxydibenzylether, Octadecyl-4-hydroxy-3,5-dimethylbenzyl-mercaptoacetat, Tris-(3,5-di-t-butyl-4-hydroxybenzyl)-amin, Bis-(4-t-butyl-3-hydroxy-2,6-dimethylbenzyl)-di-thioterephthalat, Bis-(3,5-di-t-butyl-4-hydroxybenzyl)-sulfid, Isooctyl-3,5-di-t-butyl-4-hydroxybenzyl-mercaptoacetat.
   1.7 Hydroxybenzylierte Malonate, beispielsweise Dioctadecyl-2,2-bis-(3,5-di-t-butyl-2-hydroxybenzyl)-malonat, Di-octadecyl-2-(3-t-butyl-4-hydroxy-5-methylbenzyl)-malonat, Didodecylmercaptoethyl-2,2-bis-(3,5-di-t-butyl-4-hydroxybenzyl)-malonat.
   1.8 Hydroxybenzyl-Aromaten, beispielsweise 1,3,5-Tris-(3,5-di-t-butyl-4-hydroxybenzyl)-2,4,6-trimethylbenzol, 1,4-Bis-(3,5-di-t-butyl-4-hydroxybenzyl)-2,3,5,6-tetramethylbenzol, 2,4,6-Tris-(3,5-di-t-butyl-4-hydroxybenzyl)-phenol.
   1.9 Triazinverbindungen, beispielsweise 2,4-Bis-octylmercapto-6-(3,5-di-t-butyl-4-hydroxyanilino)-1,3,5-triazin, 2-Octylmercapto-4,6-bis-(3,5-di-t-butyl-4-hydroxyanilino)-1,3,5-triazin, 2-Octylmercapto-4,6-bis-(3,5-di-t-butyl-4-hydroxyphenoxy)-1,3,5-triazin, 2,4,6-Tris-(3,5-di-t-butyl-4-hydroxyphenoxy)-1,2,3-triazin, 1,3,5-Tris-(3,5-t-butyl-4-hydroxybenzyl)-isocyanurat, 1,3,5-Tris-(4-t-butyl-3-hydroxy-2,6-dimethylbenzyl)-isocyanurat, 2,4,6-Tris-(3,5-di-t-butyl-4-hydroxyphenylethyl)-1,3,5-triazin, 1,3,5-Tris-(3,5-di-t-butyl-4-hydroxyphenylpropionyl)-hexahydro-1,3,5-triazin, 1,3,5-Tris-(3,5-dicyclohexyl-4-hydroxybenzyl)-isocyanurat.
   1.10 Benzylphosphonate, beispielsweise Dimethyl-2,5-di-t-butyl-4-hydroxybenzylphosphonat, Diethyl-3,5-di-t-butyl-4-hydroxybenzylphosphonat, Dioctadecyl-3,5-di-t-butyl-4-hydroxybenzylphosphonat, Dioctadecyl-5-t-butyl-4-hydroxy-3-methylbenzylphosphonat, Ca-Salz des 3,5-di-t-butyl-4-hydroxybenzyl-phosphonsäuremonoethylesters.
   1.11 Acylaminophenole, beispielsweise 4-Hydroxy-laurinsäureanilid, 4-Hydroxy-stearinsäureanilid, N-(3,5-Di-t-butyl-4-hydroxyphenyl)-carbaminsäureoctylester.
   1.12 Ester der β-(3,5-Di-t-butyl-4-hydroxyphenyl)-propionsäure mit ein-oder mehrwertigen Alkoholen, wie mit Methanol, Ethanol, Octadecanol, 1,6-Hexandiol, 1,9-Nonandiol, Ethylenglykol, 1,2-Propandiol, Neopentylglykol, Thiodiethylenglykol, Diethylenglykol, Triethylenglykol, Pentaerythrit, Tris-(hydroxyethyl)-isocyanurat,N,N'-Bis-(hydroxyethyl)-oxalsäurediamid, 3-Thia-undecanol, 3-Thia-pentadecanol, Trimethylhexandiol, Trimethylolpropan, 4-Hydroxymethyl-1-phospha-2,6,7-trioxabicyclo-[2,2,2]-octan.
   1.13 Ester der β-(5-t-Butyl-4-hydroxy-3-methylphenyl)-propionsäure mit ein- oder mehrwertigen Alkoholen, wie beispielsweise mit Methanol, Ethanol, Octadecanol, 1,6-Hexandiol, 1,9-Nonandiol, Ethylenglykol, 1,2-Propandiol, Neopentylglykol, Thiodietyhlenglykol, Diethylenglykol, Triethylenglykol, Pentaerythrit, Tris-(hydroxy)ethyl-isocyanurat,N,N'-Bis-(hydroxyethyl)-oxalsäurediamid,3-Thia-undecanol,3-Thiapentadecanol, Trimethylhexandiol, Trimethylolpropan, 4-Hydroxymethyl-1-phospha-2,6,7-trioxabicyclo-[2,2,2]-octan.
   1.14 Ester der β-(3,5-Dicyclohexyl-4-hydroxyphenyl)-propionsäure mit ein-oder mehrwertigen Alkoholen, wie beispielsweise Methanol, Ethanol, Octadecanol, 1,6-Hexandiol, 1,9-Nonandiol, Ethylenglykol, 1,2-Propandiol, Neopentylglykol, Thiodiethylenglykol, Diethylenglykol, Triethylenglykol, Pentaerythrit, Tris(hydroxy)ethyl-isocyanurat, N,N'-Bis-(hydroxyethyl)-oxalsäurediamid, 3-Thia-undecanol, 3-Thia-pentadecanol, Trimethylhexandiol, Trimethylolpropan, 4-Hydroxymethyl-1-phospha-2,6,7-trioxabicyclo-(2,2,2)-octan.
   1.15 Ester der 3,5-Di-t-butyl-4-hydroxyphenylessigsäure mit ein- oder mehrwertigen Alkoholen, wie beispielsweise mit Methanol, Ethanol, Octadecanol, 1,6-Hexandiol, 1,9-Nonandiol, Ethylenglykol, 1,2-Propandiol, Neopentylglykol, Thiodiethylenglykol, Diethylenglykol, Triethylenglykol, Pentaerythrit, Tris-(hydroxy)ethyl-isocyanurat,N,N'-Bis-(hydroxyethyl)-oxalsäurediamd, 3-Thia-undecanol, 3-Thia-pentadecanol, Trimethylhexandiol, Trimethylolpropan, 4-Hydroxymethyl-1-phospha-2,6,7-trioxabicyclo-[2,2,2]-octan.
   1.16 Ester der 3,3-Bis-(3'-t-butyl-4'-hydroxyphenyl)-buttersäure mit ein-oder mehrwertigen Alkoholen, wie beispielsweise mit Methanol, Ethanol, Octadecanol, 1,6-Hexandiol, 1,9-Nonandiol, Ethylenglykol, 1,2-Propandiol, Neopentylglykol, Thiodietyhlenglykol, Diethylenglykol, Triethylenglykol, Pentaerythrit, Tris-(hydroxy)ethyl-isocyanurat,N,N'-Bis-(hydroxyethyl)-oxalsäurediamid,3-Thia-undecanol,3-Thia-pentadecanol, Trimethylhexandiol, Trimethylolpropan, 4-Hydroxymethyl-1-phospha-2,6,7-trioxabicyclo-[2,2,2]-octan.
   1.17 Amide der β-(3,5-Di-t-butyl-4-hydroxyphenyl)-propionsäure, wie beispielsweise N,N'-Bis-(3,5-di-t-butyl-4-hydroxyphenylpropionyl)-hexamethylendiamin, N,N' -Bis-(3,5-di-t-butyl-4-hydroxyphenylpropionyl)-trimethylendiamn, N,N'-Bis-(3,5-di-t-butyl-4-hydroxyphenylpropionyl)-hydrazin.
2. UV-Absorber und Lichtschutzmittel
   2.1 2-(2'-Hydroxyphenyl)-benztriazole, wie beispielsweise 2-(2'-Hydroxy-5'-methylphenyl)-benztriazol, 2-(3',5'-Di-t-butyl-2'-hydroxyphenyl)-benztriazol, 2-(5'-t-Butyl-2'-hydroxyphenyl)-benztriazol, 2-[2'-Hydroxy-5'-(1,1,3,3-tetramethylbutyl)phenyl]-benztriazol, 2-(3',5'-Di-t-butyl-2'-hydroxypheny)-5-chlor-benztriazol,2-(3'-t-Butyl-2'-hydroxy-5'-methylphenyl)-5-chlor-benztriazol,2-(3'-sec-Butyl-5'-t-butyl-2'-hydroxyphenyl)-benztriazol, 2-(2'-Hydroxy-4'-octoxyphenyl)-benztriazol, 2-(3',5'-Di-t-amyl-2'-hydroxyphenyl)-benztriazol, 2-(3',5'-Bis-(α,α-dimethylbenzyl)-2'-hydroxyphenyl)-benztriazol, Mischung aus 2-(3'-t-Butyl-2'-hydroxy-5'-(2-octyloxycarbonylethyl)-phenyl)-5-chlor-benztriazol, 2-(3'-t-Butyl-5'-[2-(2-ethylhexyloxy)-carbonylethyl]-2'-hydroxyphenyl)-5-chlor-benztriazol, 2-(3'-t-Butyl-2'-hydroxy-5'-(2-methoxycarbonylethyl)phenyl)-5-chlor-benztriazol, 2-(3'-t-Butyl-2'-hydroxy-5'-(2-methoxycarbonylethyl)phenyl)-benztriazol, 2-(3'-t-Butyl-2'-hydroxy-5'-(2-octyloxy-carbonylethyl)phenyl)-benztriazol,2-(3'-t-Butyl-5'-[2-(2-ethylhexyloxy)-carbonylethyl]-2'-hydroxyphenyl)-benztriazol,2-(3'-Dodecyl-2'-hydroxy-5'-methylphenyl)-benztriazol, und 2-(3'-t-Butyl-2'-hydroxy-5'-(2-isooctylocy-carbonylethyl)phenyl-benztriazol,2,2'-Methylen-bis-[4-(1,1,3,3-tetramethylbutyl)-6-benztriazol-2-yl-phenol]; Umesterungsprodukt von 2-[3'-t-Butyl-5'(2-methoxy-carbonylethyl)-2'-hydroxy-phenyl]-benztriazol mit Polyethylenglykol 300.
   2.2 2-Hydroxybenzophenone, wie beispielsweise das 4-Hydroxy-, 4-Methoxy-, 4-Octoxy, 4-Decyloxy, 4-Dodecyloxy-, 4-Benzyloxy-, 4,2',4'-Trihydroxy-, 2'-Hydroxy-4,4'-dimethoxy-Derivat.
   2.3 Ester von gegebenenfalls substituierten Benzoesäuren, wie beispielsweise 4-t-Butyl-phenylsalicylat, Phenylsalicylat, Octylphenyl-salicylat, Dibenzoylresorcin, Bis-(4-t-butylbenzoyl)-resorcin,Benzoylresorcin,3,5-Di-t-butyl-4-hydroxybenzoesäure-2,4-di-t-butylphenylester, 3,5-Di-t-butyl-4-hydroxybenzosäure-hexadecylester, 3,5-Di-t-butyl-4-hydroxybenzoesäure-octadecylester, 3,5-Di-t-butyl-4-hydroxybenzoesäure-2-methyl-4,6-di-t-butylphenylester.
   2.4 Acrylate wie beispielsweise α-Cyan-β,β-diphenylacrylsäure-ethylester oder - isooctylester, α-Carbomethoxy-zimtsäuremethylester, α-Cyano-β-methyl-p-methoxyzimtsäuremethylester oder -butylester, α-Carbomethoxy-p-methoxy-zimtsäuremethylester, N-(β-Carbomethoxy-β-cyanovinyl)-2-methyl-indolin.
   2.5 Nickelverbindungen wie beispielsweise Nickelkomplexe des 2,2'-Thio-bis-[4-(1,1,3,3 tetramethylbutyl)-phenols] wie der 1:1 oder der 1:2 Komplex, gegebenenfalls mit zusätzlichen Liganden, wie n-Butylamin, Triethanolamin oder N-Cyclohexyldiethanolamin, Nickeldibutyldithiocarbamat, Nickelsalze von 4-Hydroxy-3,5-di-t-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6 Sterisch gehinderte Amine, wie beispielsweise Bis-(2,2,6,6-tetramethyl-piperidyl)-sebacat, Bis-(2,2,6,6-tetramethyl-piperidyl)-glutarat, Bis-(2,2,6,6-tetramethylpiperidyl)-succinat, Bis-(1,2,2,6,6-pentamethylpiperidyl)-sebacat, Bis-(1,2,2,6,6-pentamethylpiperidyl)-glutarat, Bis-(1,2,2,6,6-pentamethylpiperidyl)-sebacat,n-Butyl-3,5-di-t-butyl-4-hydroxybenzyl-malonsäure-bis(1,2,2,6,6-pentamethylpiperidyl)-ester, 2,2,6,6-Tetramethylpiperidylbehenat, 1,2,2,6,6-Pentamethylpiperidylbehenat, Kondensationsprodukt aus 1-Hydroxyethyl-2,2,6,6-tetramethyl-4-hydroxypiperidin und Bernsteinsäure, Kondensationsproduktaus N,N'-Bis-(2,2,6,6-tetramethyl-4-piperidyl)-hexamethylendiamin und 4-t-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethyl-piperidin, 4-Stearyloxy-2,2,6,6-tetramethylpiperidin, 4-Stearoyloxy-2,2,6,6-tetramethylpiperidin, 4-Stearyloxy-1,2,2,6,6-pentamethylpiperidin, 4-Stearoyloxy-1,2,2,6,6-pentamethylpiperidin, Bis-(1,2,2,6,6-pentamethylpiperidyl)-2-n-butyl-2-(2-hydroxy-3,5-di-t-butylbenzyl)-malonat, Bis-(1,2,2,6,6-pentamethylpiperidyl)-2-n-butyl-2-(4-hydroxy-3,5-di-t-butylbenzyl)-malonat, 3-n-Octyl-7,7,9,9-tetramethyl-1,3,8-triazaspiro[4,5]decan-2,4-dion, Bis-(1-octyloxy-2,2,6,6-tetramethyl-piperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylen-diamin und 4-Morpholino-2,6-dichlor-1,3,5-triazin, Kondensationsprodukt aus 2-Chlor-4,6-di-(4-n-butylamino-2,2,6,6-tetramethyl-piperidyl)-1,3,5-triazinund 1,2-Bis-(3-aminopropylamino)äthan,Kondensationsprodukt aus 2-Chlor-4,6-di-(4-methoxy-propylamino-2,2,6,6-tetramethylpiperidyl)-1,3,5-triazin und 1,2-Bis-(3-amino-propylamino)äthan,Kondensationsproduktaus 2-Chlor-4,6-di-(4-methoxypropyl-amino-1,2,2,6,6-pentamethylpiperidyl)-1,3,5-triazin und 1,2-Bis-(3-aminopropyl-amino)ethan, Kondensationsproduktaus 2-Chlor-4,6-di-(4-n-butylamino-1,2,2,6,6-pentamethylpiperidyl)-1,3,5-triazin und 1,2-Bis-(3-aminopropylamino)-ethan, Umsetzungsprodukte von 2-Chlor-4,6-di-(4-n-butylamino-2,2,6,6-tetramethylpiperidyl)-1,3,5-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-amino-propylamino)äthan, Umsetzungsprodukte von 2-Chlor-4,6-di-(4-n-butylamino-1,2,2,6,6-pentamethylpiperidyl)-1,3,5-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)äthan, Umsetzungsprodukte von 2-Chlor-4,6-di-(4-n-butyl-amino-2,2,6,6-tetramethylpiperidyl)-1,3,5-triazin und 4-t-Octylamino-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropyl-amino)ethan, Umsetzungsprodukte von 2-Chlor-4,6-di-(4-n-butylamino-1,2,2,6,6-pentamethylpiperidyl)-1,3,5-triazin und 4-t-Octylamino-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Umsetzungsprodukte von 2-Chlor-4,6-di-(4-n-butylamino-2,2,6,6-tetramethylpiperidyl)-1,3,5-triazin und 4-(4-n-Butylamino-2,2,6,6-tetramethylpiperidyl)-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamn, Triethylentetramin, Hexamethylendiamin, 1,2-Bis-(3-aminopropylamino)ethan, Umsetzungsprodukte von 2-Chlor-4,6-di-(4-n-butylamino-1,2,2,6,6-pentamethylpiperidyl)-1,3,5-triazin und 4-(4-n-Butylamino-2,2,6,6-tetramethylpiperidyl)-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, 3-Dodecyl-7,7,9,9-tetramethyl-1,3,8-triazaspiro-[4,5]-decan-2,4-dion, Oligomerisiertes 2,2,4,4-Tetramethyl-20-(oxiranylmethyl)-7-oxa-3,20-diaza-dispiro-[5.1.11.2]-heneicosan-21-on, Oligomerisiertes 1,2,2,4,4-Pentamethyl-20-(oxiranylmethyl)-7-oxa-3,20-diazadispiro-[5.1.11.2]-heneicosan-21-on, Oligomerisiertes 1-Acetyl-2,2,4,4-tetramethyl-20-(oxiranylmethyl)-7-oxa-3,20-diaza-dispiro-[5.1.11.2]-heneicosan-21-on, Dodecyl-1-(2,2,4,4-tetramethyl-4-piperidyl)pyrrolidin-2,5-dion, 3-Dodecyl-1-(1,2,2,6,6-pentamethyl-4-piperidyl)-pyrrolidin-2,5-dion, 2,2,4,4-Tetramethyl-7-oxa-3,20-diazadispiro-[5.1.11.2]-heneicosan-21-on, 2,2,4,4-Tetramethyl-7-oxa-21-oxo-3,20-diazadispiro-[5.1.11.2]-heneicosan-3-propansäure-dodecylester, 2,2,4,4-Tetramethyl-7-oxa-21-oxo-3,20-diaza-dispiro-[5.1.11.2]-heneicosan-3-propansäure-tetradecylester, 2,2,3,4,4-Pentamethyl-7-oxa-3,20-diaza-dispiro-[5.1.11.2]-heneicosan-21-on, 2,2,3,4,4-Pentamethyl-7-oxa-21-oxo-3,20-diaza-dispiro-[5.1.11.2]-heneicosan-3-propansäure-dodecylesfer, 2,2,3,4,4-Pentamethyl-7-oxa-21-oxo-3,20-diaza-dispiro-[5.1.11.2]-heneicosan-3-propansäure-tetradecylester, 3-Acetyl-2,2,4,4-tetramethyl-7-oxa-3,20-diaza-dispiro-[5.1.11.2]-heneicosan-21-on,3-Acetyl-2,2,4,4-tetramethyl-7-oxa-21-oxo-3,20-diaza-dispiro-[5.1.11.2]-heneicosan-3-propansäure-dodecylester,3-Acetyl-2,2,4,4-tetramethyl-7-oxa-21-oxo-3,20-diaza-dispiro-[5.1.11.2]-heneicosan-3-propansäure-tetradecylester, 1,1',3,3',5,5'-Hexahydro-2,2',4,4',6,6'-hexaaza-2,2',6,6'-bismethano-7,8-dioxo-4,4'-bis-(1,2,2,6,6-pentamethyl-4-piperidyl)-biphenyl, Poly-N,N'-bis-(2,2,6,6-tetramethyl-4-piperidy)-1,8-diazadecyclen, Additionsverbindung von 2,2,6,6-Tetramethyl-4-allyloxy-piperidin und Polymethylhydrogensiloxan (Molmasse bis 4000), Additionsverbindung von 1,2,2,6,6,-Pentamethyl-4-allyloxy-piperidin und Polymethylhydrogensiloxan (Molmasse bis 4000), N,N'-Di-formyl-N,N'-bis-(2,2,6,6-tetramethyl-4-piperidinyl)-hexamethylendiamin, N,N'-Di-formyl-N,N'-bis-(1,2,2,6,6-pentamethyl-4-piperidinyl)-hexamethylendiamin, 5,11-Bis-(2,2,6,6-tetramethyl-4-piperidinyl)-3,5,7,9,11,13-hexaaza-tetracyclo-[7.4.0.0^{2,7}.1^{3,13}]-tetradecan-8,14-dion,5,11-Bis-(1,2,2,6,6-pentamethyl-4-piperidinyl)-3,5,7,9,11,13-hexaaza-tetracyclo-[7.4.0.0^{2,7}.1^{3,13}]-tetradecan-8,14-dion, 7,7,9,9-Tetramethyl-8-acetyl-3-dodecyl-1,3,8-triaza-spiro-[4.5]-decan-2,4-dion, [(4-Methoxyphenyl)-methylen]-propandisäure-bis-(2,2,6,6-tetramethyl-4-piperidinyl)-ester, [(4-Methoxyphenyl)-methylen]-propandisäure-bis-(1,2,2,6,6-pentamethyl-4-piperidinyl)-ester, 2,4,6-Tris-(N-cyclohexyl-N-[2-(3,3,4,5,5-pentamethyl-piperazinon-1-yl)-ethyl]-amino)-1,3,5-triazin, Copolymerisat aus Styrol mit α-Methylstyrol und Maleinsäureanhydrid umgesetzt mit 4-Amino-2,2,6,6-tetramethylpiperidin und Octadecylamin, Copolymerisat aus Styrol mit α-Methylstyrol und Maleinsäureanhydrid umgesetzt mit 4-Amino-1,2,2,6,6-pentamethylpiperidin und Octadecylamin, Polycarbonat mit 2,2'-[(2,2,6,6-Tetramethyl-4-piperidinyl)-imino]-bis-[ethanol] als Diolkomponente,Polycarbonataus 2,2'-[(1,2,2,6,6-Pentamethyl-4-piperidinyl]-imino]-bis-[ethanol] als Diolkomponente, Copolymer aus Maleinsäureanhydrid und einem α-Olefin bis C₃₀ umgesetzt mit 4-Amino-2,2,6,6-tetramethylpiperidin, Copolymer aus Maleinsäureanhydrid und einem α-Olefin bis C₃₀ umgesetzt mit 1-Acetyl-4-amino-2,2,6,6-tetramethylpiperidin, Copolymer aus Maleinsäureanhydrid und einem α-Olefin bis C₃₀ umgesetzt mit 4-Amino-1,2,2,6,6-pentamethylpiperidin, sowie die N-Alkyl- und N-Aryloxyderivate der oben genannten Verbindungen mit freien NH-Gruppen am Piperidin, speziell α-Methylbenzyloxy- und Alkyloxy- von C₁ bis C₁₈.
   2.7 Oxalsäurediamide, wie beispielsweise 4,4'-Di-octyloxy-oxanilid, 2,2'-Di-octyloxy-5,5'-di-t-butyl-oxanilid,2,2'-Di-dodecyloxy-5,5'-di-t-butyl-oxanilid,2-Ethoxy-2'-ethyl-oxanilid, N,N'-Bis-(3-dimethylaminopropyl)-oxalamid, 2-Ethoxy-5-t-butyl-2'-ethyloxanilid und dessen Gemisch mit 2-Ethoxy-2'-ethyl-5,4'-di-t-butyl-oxanilid, Gemische von o- und p-Methoxy- sowie von o- und p-Ethoxy-di-substituierten Oxaniliden.
   2.8 2-(2-Hydroxyphenyl)-1,3,5-triazine, wie beispielsweise 2,4,6-Tris-(2-hydroxy-4-octyloxyphenyl)-1,3,5-triazin, 2-(2-Hydroxy-4-octyloxyphenyl)-4,6-bis-(2,4-dimethylphenyl)-1,3,5-triazin,2-(2,4-Dihydroxyphenyl)-4,6-bis-(2,4-dimethyl-phenyl)-1,3,5-triazin,2-(2,4-Dihydroxyphenyl)-4,6-bis-(2,4-dimethylphenyl)-1,3,5-triazin,2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-di-methylpheny)-1,3,5-triazin, 2-[2-Hydroxy-4-(2-hydroxy-3-butyloxy-propyloxy)phenyl]-4,6-bis(2,4-dimethylpheny)-1,3,5-triazin,2-[2-hydroxy-4-(2-hydroxy-3-octyloxy-propyloxy)phenyl]-4,6-bis(2,4-dimethyphenyl)-1,3,5-triazin.
3. Metalldesaktivatoren, wiebeispielsweise N,N'-Diphenyloxalsäurediamid, N-Salicyal-N'-salicyloylhydrazin, N,N'-Bis-(salicyloyl)-hydrazin, N,N'-Bis-(3,5-di-t-butyl-4-hydroxyphenylpropionyl)-hydrazin, 3-Salicyloylamino-1,2,4-triazol, Bis-(benzyliden)oxalsäuredihydrazid, Oxanilid, Isophthalsäure-dihydrazid, Sebacinsäure-bis-phenylhydrazid, N,N'-Diacetyl-adipinsäure-dihydrazd, N,N'-bis-salicyloyl-oxalsäuredihydrazid, N,N'-Bis-salicyloyl-thiopropionsäure-dihydrazid.
4. Phosphite und Phosphonite, wie beispielsweise Triphenylphosphit, Diphenylalkylphosphite, Phenyldialkylphosphite, Tris-(nonylphenyl)-phosphit, Trilaurylphosphit,Trioctadecylphosphit, Distearyl-pentaerythritfdiphosphit, Tris-(2,4-di-t-butylphenyl)-phosphit,Diisodecylpentaerythrit-diphosphit,Bis-(2,4-di-t-butylphenyl)-pentaerythritdiphosphit, Bis-(2,6-di-t-butyl-4-methylphenyl)-pentaerythritdiphosphit, Bis-isodecyloxy-pentaerythritdiphosphit, Bis-(2,4-di-t-butyl-6-methylphenyl)-pentaerythritdiphosphit, Bis-(2,4,6-tri-t-butylphenyl)-pentaerytritdiphosphit, Tristearylsorbit-triphosphit, Tetrakis-(2,4-di-t-butylphenyl)-4,4'-biphenylen-diphosphonit, 6-Isooctyloxy-2,4,8,10-tetra-t-butyl-12H-dibenz[d,g]-1,3,2-dioxaphosphocin, 6-Fluor-2,4,8,10-tetra-t-butyl-12-methyl-dibenz-[d,g]-1,3,2-dioxaphophocin, Bis-(2,4-di-t-butyl-6-methylphenyl)-methylphosphit, Bis-(2,4-di-t-butyl-6-methylphenyl)-ethylphosphit, Tris(2-t-butyl-4-thio-(2'-methenyl-4'-hydroxy-5'-t-butyl)-pheny-5-methenyl)-phenylphosphit.
5. Peroxidzerstörende Verbindungen wie beispielsweise Ester der β-Thiodipropionsäure, beispielsweise der Lauryl-, Stearyl-, Myristyl- oder Tridecylester, Mecaptobenzimidazol, das Zinksalz des 2-Mercaptobenzimidazols, Zink-alkyldithiocarbamate, Zinkdibutyl-dithiocarbamat, Dioctadecylmonosulfid, Dioctadecyldisulfid, Pentaerytrit-tetrakis-(β-dodecylmercapto)-propionat.
6. Polyamidstabilisatoren, wie beispielsweise Kupfersalze in Kombination mit Jodiden und/oder Phosphorverbindungen und Salze des zweiwertigen Mangans.
7. Basische Co-Stabilisatoren wie beispielsweise Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie beispielsweise 4-t-Butylbenzoesäure, Adipinsäure, Diphenylessigsäure.
9. Füllstoffe und Verstärkungsmittel, wie beispielsweise Calciumcarbonat, Silikate, Glasfasern, Asbest, Talk, Kaolin, Glimmer, Bariumsulfat, Metalloxide und - hydroxide, Ruß, Graphit.
10. Sonstige Zusätze, wie beispielsweise Weichmacher, Gleitmittel, Emulgatoren, Pigmente, Optische Aufheller, Flammschutzmittel, Antistatika, Treibmittel.

Die Additive werden nach allgemein üblichen Methoden in die organischen Polymeren eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglichem Verdunstenlassen des Lösemittels kann die Einarbeitung erfolgen. Die Verbindungen sind auch wirksam, wenn sie in ein bereits granuliertes Polymer nachträglich in einem gesonderten Verarbeitungsschritt eingebracht werden.

Die erfindungsgemäß zu verwendenden Verbindungen können auch in Form eines Masterbatches, der diese Verbindungen beispielsweise in einer Konzentration von 1 bis 75, vorzugsweise 2,5 bis 30 Gew.-% enthält, den zu stabilisierenden Polymeren zugesetzt werden.

Die nachfolgenden Beispiele sollen die Erfindung näher erläutern.

Zur Charakterisierung der nicht kristallisierenden Verbindungen eignet sich besonders die Verschiebung von Signalen im ¹H-NMR. Bei der Umsetzung der Alkohol-Funktion am 2-Hydroxy-1,3-propandiyl-Strukturelementverschiebt sich das ¹H-NMR-Signal am C² deutlich (-C¹H₂-C²H(OH)-C³H₂-). Zur Charakterisierung der Verbindungen wird entweder die Verschiebung dieses ¹H-NMR-Signals, oder ein anderes charakteristisches Signal im ¹H-NMR angegeben.

### Beispiel 1

### 20,20'-(2-Hydroxy-1,3-propandiyl)-bis-[3-(1'-methylbenzyloxy)-2,2,4,4-tetramethyl-7-oxa-3,20-diazadispiro-[5.1.11.2]-heneicosan-21-on]

78,3 g 20,20'-(2-Hydroxy-1,3-propandiyl)-bis-[2,2,4,4-tetramethyl-7-oxa-3,20-diazadispiro-[5.1.11.2]-heneicosan-21-on] und 3,0g Molybdäntrioxyd wurden in 500 cm³ Ethylbenzol vorgelegt. Unter Schutzgas wurden bei 110°C 96,6 g einer 70%igen t-Butylhydroperoxidlösung langsam zugetropft. Danach wurde das gesamte Wasser durch azeotrope Destillation entfernt und das Gemisch noch weitere 8 h zum Sieden erhitzt. Das Molybdäntrioxid wurde abfiltriert, überschüssiges Peroxid zerstört und die Lösung mit Wasser und verdünnter Salzsäure ausgeschüttelt. Nach dem Trocknen wurde das Ethylbenzol abdestilliert; es blieben 100,1 g der gesuchten Verbindung als hellgelbes Harz zurück. Im ¹H-NMR erschien das >N-O-CH- Signal als Quartett bei etwa 4,75 ppm bezüglich TMS.

### Beispiel 2

### 20,20'-(2-Hydroxy-1,3-propandiyl)-bis-[3-octyloxy-2,2,4,4-tetramethyl-7-oxa-3,20-diazadispiro-[5.1.11.2]-heneicosan-21-on]

Die Durchführung erfolgt wie unter Beispiel 1 beschrieben, jedoch wurde n-Octan anstatt Ethylbenzol eingesetzt. Nach dem Abdestillieren blieben 101,1 g des Produkts als schwach gelbe, glasartige Masse zurück. Die >N-O-CH- Atome kamen im 1H-NMR schlecht aufgelöst bei 3,4 bis 3,9 ppm bezüglich TMS.

### Beispiel 3

### 20,20'-(2-Hydroxy-1,3-propandiyl]-bis-[3-(1'-methylbenzyloxy)-2,2,4,4-tetramethyl-7-oxa-3,20-diazadispiro-[5.1.11.2]-heneicosan-21-on]

48,5 g 3-(1'-Methylbenzyloxy)-2,2,4,4-pentamethyl-7-oxa-3,20-diazadispiro-[5.1.11.2]-heneicosan-21-on wurden in 125 cm³ Toluol bei 70°C vorgelegt und 4,0 g 50%ige Natronlauge zugegeben. Danach wurde die Temperatur auf 90°C erhöht, 4,6 g Epichlorhydrin und anschließend weitere 36 g 50%ige Natronlauge zugegeben. Die Reaktionsmischung wurde 8 h zum Sieden erhitzt, mit 150 cm³ Wasser versetzt und eine weitere Stunde erhitzt. Noch zweimal wurden je 150 cm³ Wasser zugegeben und der Ansatz danach 1 h unter Rückfluß erhitzt. Die Phasen wurden getrennt, die organische Phase mit Wasser ausgeschüttet, getrocknet und eingeengt. Zurück blieben 47,6 g einer klaren, glasartigen Masse derselben Verbindung wie unter Beispiel 1 beschrieben.

### Beispiel 4

### 20,20'-(2-Hydroxy-1,3-propandiyl)-bis-[3-octyloxy-2,2,4,4-tetramethyl-7-oxa-3,20-diazadispiro-[5.1.11.2]-heneicosan-21-on]

Die Durchführung erfolgte wie unter Beispiel 3 beschrieben, jedoch wurden für die Reaktion 49,3 g 3-Octyloxy-2,2,4,4-pentamethyl-7-oxa-3,20-diazadispiro-[5.1.11.2]-heneicosan-21-on eingesetzt. Erhalten wurden 47,0 g derselben Verbindung wie in Beispiel 2 beschrieben als gelbes Harz.

### Beispiel 5

### 20,20'-(2-Methoxy-1,3-propandiyl)-bis-[3-(1'-methylbenzyloxy)-2,2,4,4-tetramethyl-7-oxa-3,20-diazadispiro-[5.1.11.2]-heneicosan-21-on]

0,56 g Natriumhydrid wurden in 30 cm³ Toluol vorgelegt. Bei 100°C wurde eine Lösung von 20,5 g 20,20'-(2-Hydroxy-1,3-propandiyl)-bis-[3-(1'-methylbenzyloxy)-2,2,4,4-tetramethyl-7-oxa-3,20-diazadispiro-[5.1.11.2]-heneicosan-21-on] in 50 cm³ Toluol langsam zugetropft und das Gemisch 1 h gerührt. 2,8 g Dimethylsulfat in 20 cm³ Toluol wurden ebenfalls bei dieser Temperatur zugetropft und der Ansatz weitere 5 h zum Sieden erhitzt. Danach wurde der Ansatz auf Wasser gegossen, die organische Phase abgetrennt und mit Wasser ausgeschüttelt. Nach dem Trocknen und Abdestillieren des Lösemittels blieben 19,6 g des gesuchten Produkts als glasartige Masse zurück. Im ¹H-NMR trat das -O-CH₃-Signal bei etwa 3,35 ppm auf.

### Beispiel 6

### 20,20'-(2-Methoxy-1,3-propandiyl)-bis-[3-(1'-octyloxy)-2,2,4,4-tetramethyl-7-oxa-3,20-diazadispiro-[5.1.11.2]-heneicosan-21-on]

Die Durchführung erfolgte wie unter Beispiel 5 beschrieben. Anstatt der 2-Methylbenzyloxy-Verbindungwurdenjedoch 20,8 g 3-Octyloxy-2,2,4,4-tetramethyl-7-oxa-3,20-diazadispiro-[5.1.11.2]-heneicosan-21-on eingesetzt. Erhalten wurden 15,2 g eines gelbliches Harzes. Im ¹H-NMR trat das -O-CH₃-Signal bei etwa 3,4 ppm auf.

### Beispiel 7

### 20,20'-(2-Benzyloxy-1,3-propandiyl)-bis-[3-(1'-methylbenzyloxy)-2,2,4,4-tetramethyl-7-oxa-3,20-diazadispiro-[5.1.11.2]-heneicosan-21-on]

Die Durchführung erfolgte wie unter Beispiel 5 beschrieben, jedoch wurden anstatt des Dimethylsulfats 3,8 g Benzylbromid eingesetzt. Nach der Aufarbeitung blieben 19,0 g einer hellbraunen, glasartigen Masse zurück. Die benzylische -O-CH₂-Gruppe kam im ¹H-NMR bei etwa 4,5 ppm.

### Beispiel 8

### 20,20'-(2-Benzyloxy-1,3-propandiyl)-bis-[3-octyloxy-2,2,4,4-tetramethyl-7-oxa-3,20-diazadispiro-[5.1.11.2]-heneicosan-21-on]

Die Durchführung erfolgte wie unter Beispiel 7 beschrieben. Anstatt der 1'-Methylbenzyloxy-Verbindung wurden jedoch 20,8 g 20,20'-(2-Hydroxy-1,3-propandiyl)-bis-[3-octyloxy-2,2,4,4-tetramethyl-7-oxa-3,20-diazadispiro-[5.1.11.2]-heneicosan-21-on] eingesetzt. Erhalten wurden 18,8 g eines hellbraunen Produkts. Im ¹H-NMR trat das benzylische -O-CH₂-Signal bei etwa 4,5 ppm auf.

### Beispiel 9

### 20,20'-(2-Acetyloxy-1,3-propandiyl)-bis-[3-(1'-methylbenzyloxy)-2,2,4,4-tetramethyl-7-oxa-3,20-diazadispiro-[5.1.11.2]-heneicosan-21-on]

20,5 g 20,20'-(2-Hydroxy-1,3-propandiyl)-bis-[3-(1'-methylbenzyloxy)-2,2,4,4-tetramethyl-7-oxa-3,20-diazadispiro-[5.1.11.2]-heneicosan-21-on]wurdenin 100 cm³ Acetanhydrid gelöst. Während 6 h destillierte man circa 50 cm³ des Lösemittels im Gemisch mit der entstehenden Essigsäure ab. Danach engte man das Gemisch vollends im Vakuum ein. Zurück blieben 19,3 g der gesuchten Verbindung, die jedoch noch Spuren von Acetenhydrid und Eisessig enthielten. Diese lassen sich durch Auflösen in Methyl-t-butyl-ether und Ausschütteln mit verdünnter Natriumhydrogencarbonat-Lösungund Wasser entfernen. Nacherneutem Einengen des Ansatzes wurden 17,5 g der gesuchten Acetyl-Verbindung als farblose, glasartige Masse erhalten. Die Acetyl-CH₃-Gruppe erschien im ¹H-NMR bei etwa 2,0 ppm.

### Beispiel 10

### 20,20'-(2-Acetyloxy-1,3-propandiyl)-bis-[3-octyloxy-2,2,4,4-tetramethy-7-oxa-3,20-diazadispiro-[5.1.11.2]-heneicosan-21-on]

Die Durchführung erfolgte wie unter Beispiel 9 beschrieben, jedoch mit 20,8 g 20,20'-(2-Hydroxy-1,3-propandiyl)-bis-[3-octyloxy-2,2,4,4,-tetramethyl-7-oxa-3,20-diazadispiro-[5.1.11.2]-heneicosan-21-on] anstatt der 1'-Methylbenzyloxy-Verbindung. Erhalten wurden 18,6 g eines farblosen Harzes. Im ¹H-NMR trat das Acetyl-CH₃-Signal bei etwa 2,0 ppm auf.

### Beispiel 11

### 20,20'-(2-Benzoyloxy-1,3-propandiyl)-bis-[3-(1'-methylbenzyloxy)-2,2,4,4-tetramethyl-7-oxa-3,20-diazadispiro-[5.1.11.2]-heneicosan-21-on]

20,5 g 20,20'-(2-Hydroxy-1,3-propandiyl)-bis-[3-(1'-methylbenzyloxy)-2,2,4,4-tetramethyl-7-oxa-3,20-diazadispiro-[5.1.11.2]-heneicosan-21-on] wurden in 60 cm³ Toluol vorgelegt und vorsichtig mit 14 cm³ 1,6molarer Butyllithium-Lösung versetzt. Nach 1 h wurden 3,1 g Benzoylchlorid in 20 cm³ Toluol zugetropft und das Gemisch weitere 4 h gerührt. Der Ansatz wurde mit Wasser versetzt, die organische Phase mehrmals mit Wasser ausgeschüttelt, getrocknet und eingeengt. Zurück blieben 20,0 g Produkt (Signalverschiebung im ¹H-NMR von 3,9 nach circa 5,6 ppm).

### Beispiel 12

### 20,20'-(2-Benzoyloxy-1,3-propandiyl)-bis-[3-octyloxy-2,2,4,4-tetramethyl-7-oxa-3,20-diazadispiro-[5.1.11.2]-heneicosan-21-on]

Die Durchführung erfolgte wie unter Beispiel 11 beschrieben, aber mit 20,8 g 20,20'-(2-Hydroxy-1,3-propandiyl)-bis-[3-octyloxy-2,2,4,4-tetramethyl-7-oxa-3,20-diazadispiro-[5.1.11.2]-heneicosan-21-on], wobei 21,5 g hellgelbes Produkt (Verschiebung im ¹H-NMR von 3,9 nach 5,6 ppm) erhalten wurden.

Verfährt man analog den Beispiel 11 und 12, so erhält man weitere erfindungsgemäße Verbindungen, die in der nachstehenden Tabelle aufgeführt sind: für n = 2 werden nur 50 Mol-% der Komponente AZₙ bez. des Alkohols eingesetzt.

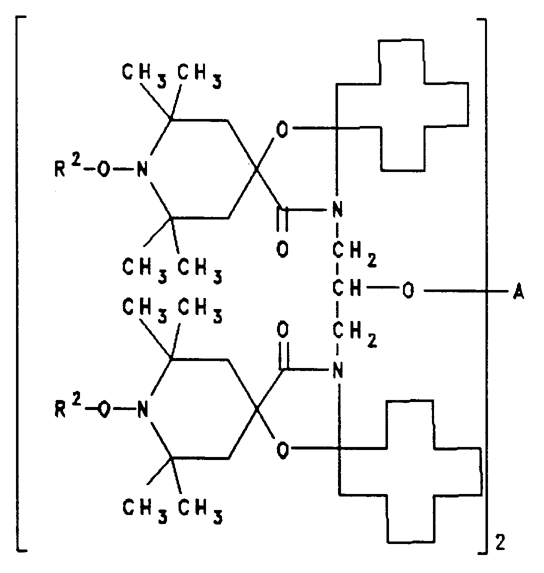

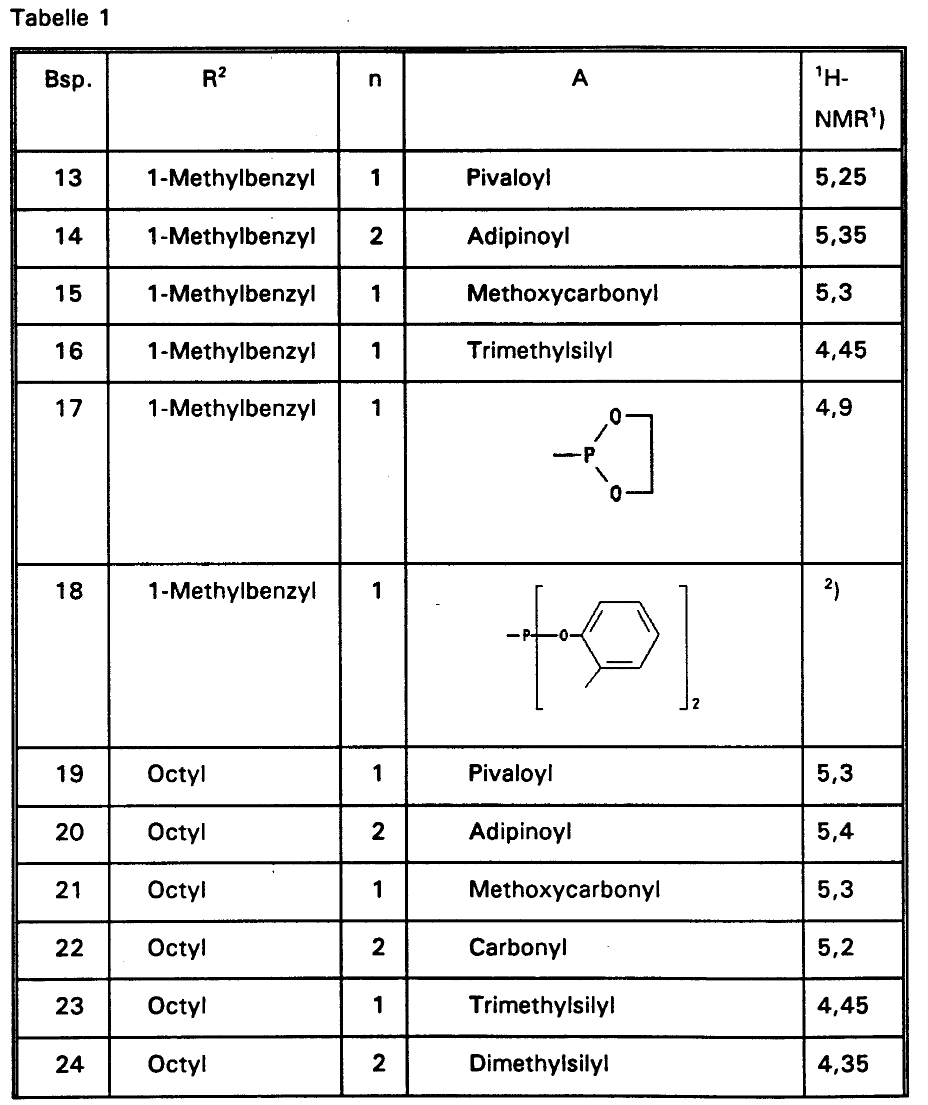

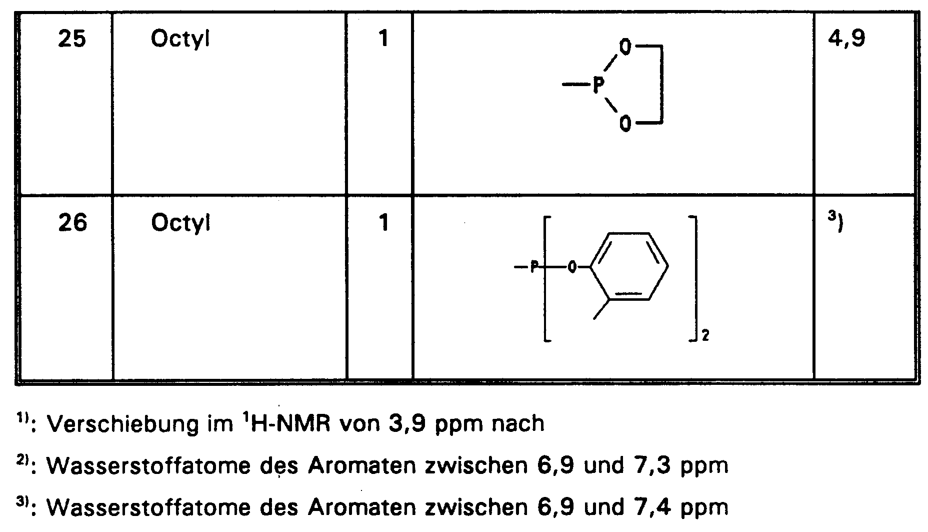

Weitere erfindungsgemäße Verbindungen werden durch Unsetzung der Alkohole mit Isocyanaten erhalten:

### Beispiel 27

15,4 g 20,20'-(2-Hydroxy-1,3-propandiyl)-bis-[3-(1'-methylbenzyloxy)-2,2,4,4-tetramethyl-7-oxa-3,20-diazadispiro-[5.1.11.2]-heneicosan-21-on] wurden in 60 cm³ Toluol gelöst und mit 2,15 g Phenylisocyanat versetzt. Anschließend wurde der Ansatz 24 h unter Rückfluß erhitzt und danach das Lösemittel im Vakuum abdestilliert. Zurück blieben 16,9 g der gesuchten Verbindung als hellgelbe, glasartige Verbindung. Im IR-Spektrum erschien die Carbonylbande der Urethan-Gruppe bei etwa 1735 cm⁻¹.

Analog Beispiel 27 lassen sich weitere erfindungsgemäße Verbindungen durch Reaktion mit Isocyanaten herstellen (Umsetzungen mit 1,6-Diisocyanatohexan im Molverhältnis 2:1):

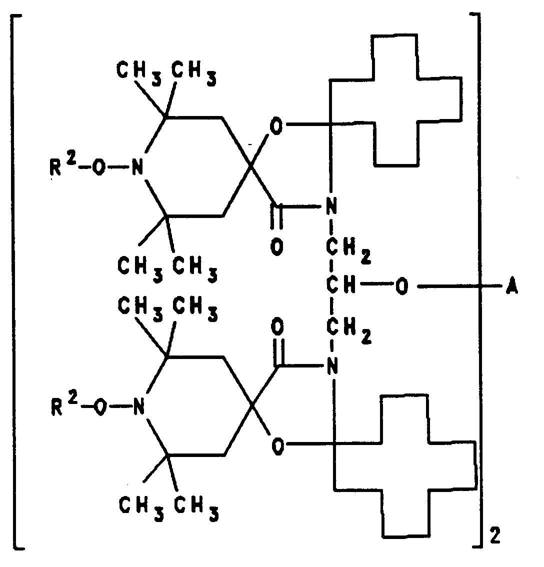

**Tabelle 2**

| Bsp. | R² | n | A' | IR¹) |
|---|---|---|---|---|
| 28 | 1-Methylbenzyl | 1 | Cyclohexyl | 1720²) |
| 29 | 1-Methylbenzyl | 2 | Hexamethylen | 1720²) |
| 30 | Octyl | 1 | Phenyl | 1735 |
| 31 | Octyl | 1 | Cyclohexyl | 1720²) |
| 32 | Octyl | 2 | Hexamethylen | 1720²) |

| | | | | |
|---|---|---|---|---|
| ¹): Charakterisierung durch die Lage der Urethan-Carbonylgruppe im IR-Spektrumⁱⁿ cm⁻¹. | | | | |
| ²): als Schulter | | | | |

### Beispiel 33

### 20,20'-(2-Acetyloxy-1,3-propandiyl)-bis-[3-acetyl-2,2,4,4-tetramethyl-7-oxa-3,20-diazadispiro-[5.1.11.2]-heneicosan-21-on]

39,2 g 20,20'-(2-Hydroxy-1,3-propandiyl)-bis-[2,2,4,4-tetramethyl-7-oxa-3,20-diazadispiro-[5.1.11.2]-heneicosan-21-on]wurden in 400 cm³ Acetanhydrid gelöst und so stark erhitzt, daß während 6 Stunden ca 200 cm³ Flüssigkeit abdestillierten. Danach wurde der Ansatz im Vakuum zur Trockene eingeengt, der Rückstand in Toluol aufgenommen, mit verdünnter Hydrogencarbonatlösung und Wasser ausgeschüttelt und die organische Phase getrocknet. Nach dem Einengen kristallisierte die gesuchte Verbindung beim Ausrühren mit Hexan als farbloser Feststoff aus. 36,9 g mit einem Schmelzpunkt von 122°C wurden erhalten.

### Beispiel 34

### 20,20'-(2-Trimethylsilyloxy-1,3-propandiyl)-bis-[3-acetyl-2,2,4,4-tetramethyl-7-oxa-3,20-diazadispiro-[5.1.11.2]-heneicosan-21-on]

Die Durchführung erfolgte wie in Beispiel 16 beschrieben. Aus 43,4 g 20,20'-(2-Hydroxy-1,3-propandiyl)-bis-[3-acetyl-2,2,4,4-tetramethyl-7-oxa-3,20-dazadispiro-[5.1.11.2]-heneicosan-21-on] und 5,9 g Chlortrimethylsilan wurden auf diese Weise 42,2 g eines gelblichen, glasartigen Produkts erhalten.

### Beispiel 35

### 20,20'-(2-Phenylaminocarbonyloxy-1,3-propandiyl)-bis-[3-acetyl-2,2,4,4-tetramethyl-7-oxa-3,20-diazadispiro[5.1.11.2]-heneicosan-21-on]

Die Durchführung erfolgte wie in Beispiel 27 beschrieben. Aus 43,4 g 20,20'-(2-Hydroxy-1,3-propandiyl)-bis-[3-acetyl-2,2,4,4-tetramethyl-7-oxa-3,20-diazadispiro-[5.1.11.2]-heneicosan-21-on] und 6,3 g Phenylisocyanat erhielt man 36,4 g der gesuchten Verbindung als blaßgelbe, glasartige Masse.

### Beispiel 36

### Löslichkeit in Hexan

| | Löslichkeit in Hexan bei Raumtemperatur |
|---|---|
| Vergl. A (EP 57 885, Bsp. 16) | ≺ 1 Gew.% |
| Bsp. 1 | ≻ 30 Gew.% |
| Bsp. 2 | ≻ 30 Gew.% |
| Bsp. 35 | ca. 5 Gew.% |

### Beispiel 37

### Lichtstabilisierende Wirkung

100 Gewichtsteile Polypropylen mit einem MFI 230/5 von 3 g/10 min und einer Dichte von 0,903 g/cm³ wurden mit 0,2 Gewichtsteilen Calciumstearat, 0,1 Gewichtsteilen Pentaerythrityl-tetrakis-3-(3,5-di-t-butyl-4-hydroxyphenyl)-propionat, 0,1 Gewichtsteilen Tris-(2,4-di-t-butylphenyl)phosphit und 0,2 Gewichtsteilen des zu prüfenden Stabilisators 5 min bei 200°C geknetet. Aus dieser Mischung wurde bei 190°C eine 150 µm starke Folie gepreßt und in einem Bewitterungsgerät (®Xenotest 1200) belichtet. Als Kriterium wurde der Abfall der Reißdehnung auf 50% gewählt.

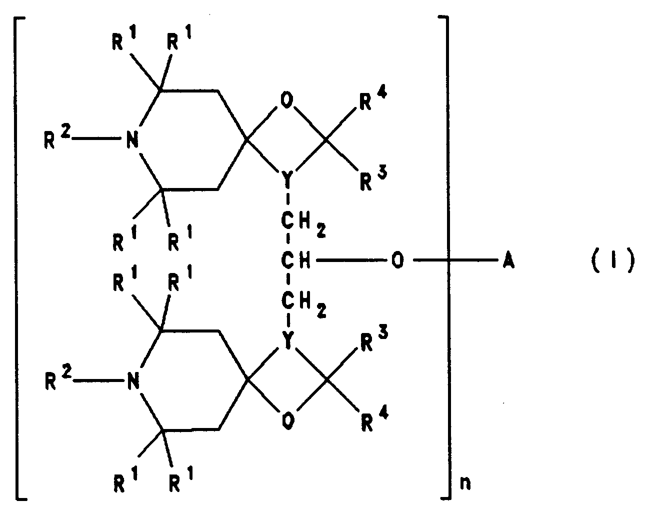

| | R² = | A = | Abfall der Reißdehnung auf 50% |
|---|---|---|---|
| Bsp. aus EP 57 885 (Vgl. B) | H | C₆H₅-NH-CO | 315 h |
| Bsp. 1 | C₆H₅-CH(CH₃)-O- | C₆H₅-NH-CO | 375 h |
| Bsp. 2 | C₈H₁₇-O- | C₆H₅-NH-CO | 390 h |
| Bsp. 35 | CH₃-CO- | C₆H₅-NH-CO | 380 h |

### Beispiel 38

### Stabilisierende Wirkung im WAB-Test

Die gleiche Mischung wie in Beispiel 37 wurde mittels eines Extruders bei 200 bis 230°C granuliert. Aus dem Granulat wurden bei 210 bis 240°C 1 mm dicke Spritzplatten hergestellt. Diese wurden bei einer Temperatur von 140°C gelagert und als Kriterium die mechanische Versprödung gemessen. Die Abkürzungen X und Y beziehen sich auf die Formel in Beispiel 37.

| | R² = | A = | mechanische Versprödung |
|---|---|---|---|
| Bsp. aus EP 57 885 (Vgl. C) | H | C₆H₅-NH-CO | 43 d |
| Bsp. 1 | C₆H₅-CH(CH₃)-O- | C₆H₅-NH-CO | 57 d |
| Bsp. 2 | C₈H₁₇-O- | C₆H₅-NH-CO | 58 d |
| Bsp. 35 | CH₃-CO- | C₆H₅-NH-CO | 61 d |

## Patentansprüche

1. Polyalkylpiperidin-Verbindungen der Formel I 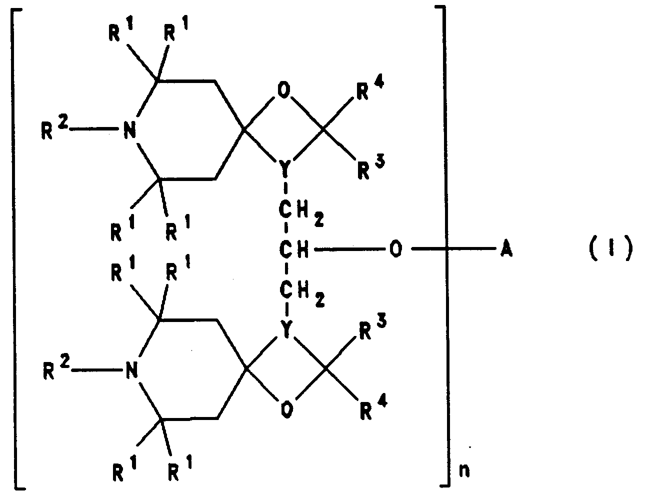 worin Y eine der Gruppen 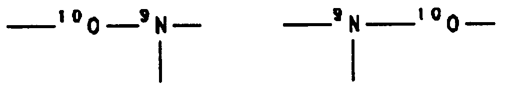 ist,
R¹ ein Wasserstoffatom oder eine Alkylgruppe bedeutet,
R² eine Gruppe der Formel R¹⁷-O- oder R¹⁸-CO- (Acyl) ist,
R³ und R⁴ gleich oder verschieden sind und ein Wasserstoffatom oder eine Alkylgruppe bedeuten, oder R³ und R⁴ zusammen mit dem sie verbindenden C-Atom einen Ring mit 5 bis 12 Ringliedern oder eine Gruppe der Formel 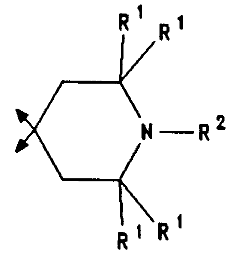 bilden,
n = 1, 2 oder 3 bedeutet und
A bei n = 1
ein Wasserstoffatom, R⁵-, R⁵-CO-, R⁵-NH-CO- (Urethan), -COOR⁵, -P(OR⁶)(OR⁷), -Si(OR⁵)(OR⁵)(OR⁵), -SiR⁵R⁵R⁵ oder die Gruppe 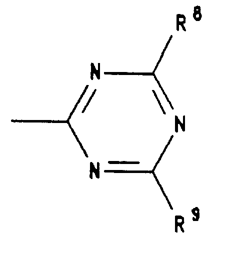 A bei n = 2
-R¹¹-, -CO-R¹²-CO-, -CO-NH-R¹³-NH-CO-, -CO-, = POR⁵, = Si(OR⁵)OR⁵), = SiR⁵R⁵ oder die Gruppe 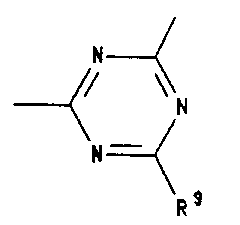 A bei n = 3
R¹⁴≡, R¹⁵(CO-)₃, R¹⁶(NH-CO-)_{3,} ≡Si(OR⁵), ≡SiR⁵ oder die Gruppe 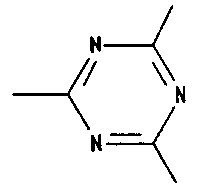 bedeutet,
R⁵ eine C₁-C₁₈-Alkylgruppe, eine C₂-C₁₈-Alkenylgruppe, eine C₂-C₁₈-Alkinylgruppe, eine C₅-C₁₂-Cycloalkylgruppe, eine C₆-C₁₀-Bicycloalkylgruppe, eine C₅-C₈-Cycloalkenylgruppe, eine C₆-C₁₀-Arylgruppe, eine C₇-C₉-Aralkylgruppe, eine C₇-C₉-Alkarylgruppe, gegebenenfalls substituiert durch C₁-C₄-Alkyl oder Phenyl, bedeutet, wobei mehrere Reste R⁵ gleich oder verschieden sein können,
R⁶ und R⁷ gleich oder verschieden sind und eine C₁-C₁₈-Alkylgruppe, eine C₂-C₁₈-Alkenylgruppe, eine C₂-C₁₈-Alkinylgruppe, eine C₅-C₁₂-Cycloalkylgruppe, eine C₆-C₁₀-Bicycloalkylgruppe, eine C₅-C₈-Cycloalkenylgruppe, eine C₆-C₁₀-Arylgruppe, eine C₇-C₉-Aralkylgruppe oder eine C₇-C₉-Aralkylgruppe, substituiert durch C₁-C₄ -Alkyl oder Phenyl, bedeuten, oder ein Rest sind der Formel II oder III 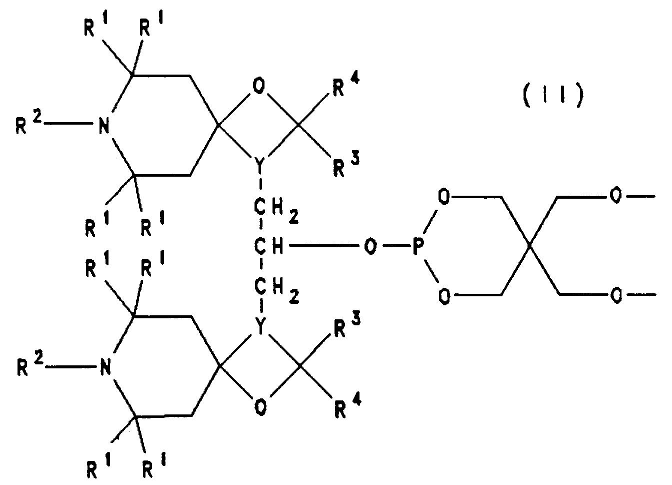 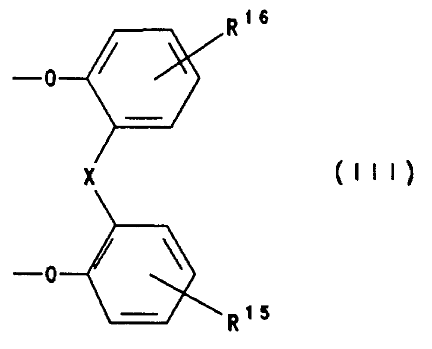 worin R¹, R², R³, R⁴ und R⁵ die obengenannte Bedeutung haben, und X eine direkte Bindung oder Methylengruppe oder eine 1,1-Alkylidengruppe mit 2 bis 5 C-Atomen bedeutet,
R⁸ und R⁹ gleich oder verschieden sind und -OR⁵, -NHR⁵, -NR¹⁷R¹⁸, oder eine der beiden Gruppen 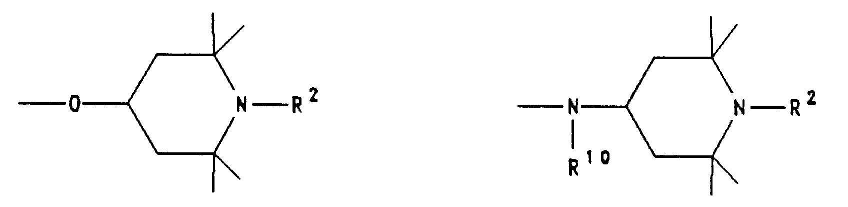 worin R¹⁰ ein Wasserstoffatom, eine C₁-C₁₂-Alkylgruppe, eine C₅-C₇-Cycloalkylgruppe, eine C₇-C₉-Aralkylgruppe, eine C₈-C₁₈-Alkanoylgruppe, eine C₃-C₅-Alkenoylgruppe oder eine Benzoylgruppe bedeutet, oder 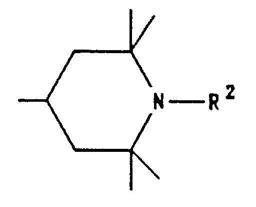 ist,
R¹¹, R¹² und R¹³ gleich oder verschieden sind und eine C₁-C₁₂-Alkylengruppe, eine C₅-C₇-Cycloalkylengruppe, eine C₈-C₁₀-Bicycloalkylengruppe, eine C₈-C₁₀-Arylengruppe, eine C₇-C₉-Aralkylengruppe oder C₇-C₉-Arylengruppe, substituiert durch C₁-C₄-Alkyl oder Phenyl, bedeuten,
R¹⁴, R¹⁵ und R¹⁶ gleich oder verschieden sind und einen dreifach funktionellen C₃-C₁₈-Alkyl-, C₅-C₇-Cycloalkyl-, C₆-C₁₀-Aryl-oder C₆-C₁₀-Arylrest, substituiert durch C₁-C₄-Alkyl oder Phenyl bedeuten,
R¹⁷ und R¹⁸ gleich oder verschieden sind und eine C₁-C₁₀-Alkylgruppe, eine C₅-C₇-Cycloalkylgruppe, eine C₆-C₁₀-Arylgruppe bedeuten, oder R¹⁷ und R¹⁸ zusammen mit dem Stickstoff einen 5- bis 12-gliedrigen Ring bilden, der auch noch Sauerstoff enthalten kann.

2. Verbindungen gemäß Anspruch 1, dadurch gekennzeichnet, daß in Formel I R¹ eine Methylgruppe ist.

3. Verbindungen gemäß Anspruch 1, dadurch gekennzeichnet, daß in Formel I R² eine C₁-C₁₈-Acylgruppe ist.

Polyalkylpiperidin-Verbindungen der Formel I 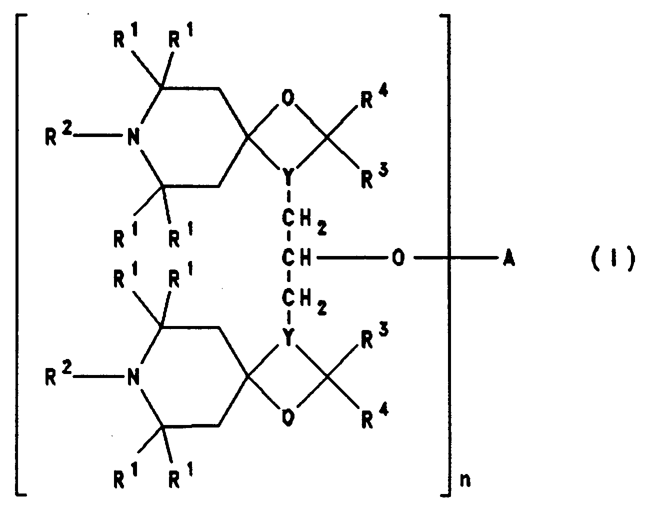 worin Y eine der Gruppen 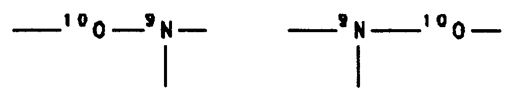 ist, sind sehr wirksame Stabilisatoren für organische Stoffe.
